# EUROPEAN PATENT APPLICATION

(11) **EP 2 338 480 A1**
(43) Date of publication of application: **29.06.2011**
(21) Application number: 09180344.5
(22) Date of filing: 22.12.2009
(51) Int. Cl.: A61K 9/50, A61K 31/192, A61K 31/501, A61K 31/519, A61K 31/7048

(54) **Coating of particles comprising a pharmaceutically active ingredient with a carbonate salt or phosphate salt**

(71) Applicant: LEK Pharmaceuticals d.d., 1526 Ljubljana (SI)
(72) Inventor: The designation of the inventor has not yet been filed

(57) **Abstract**

The present invention belongs to the field of pharmaceutical industry and relates to a process for coating a particle comprising a pharmaceutically active ingredient (API) comprising the steps of providing a composition comprising carbonate ions, phosphate ions, or a mixture thereof, providing a particle comprising an API, and precipitating a carbonate salt, a phosphate salt or a mixture thereof onto said particles.

The present invention is also directed to a particle comprising an API, wherein the particle is coated with a coating comprising a carbonate salt, phosphate salt, or mixture thereof, and to a pharmaceutical composition comprising said particles.

Moreover, the present invention is also directed to the use of said particles for preparing a medicament.

## Description

### Field of the Invention

The present invention relates to the field of pharmaceutical industry, more specifically to the field of preparing dosage forms, and relates to a process for coating a particle comprising a pharmaceutically active ingredient (API). The process comprises the steps of providing a composition comprising specific ions, providing a particle comprising an API and precipitating the salts of said ions onto said particles.

The present invention further relates to a coated particle comprising an API. Moreover, the present invention is also directed to the use of said particles.

### Description of the background art

Inorganic salts, such as calcium phosphate or calcium carbonate, build a mineral part of the bone or tooth hard tissue and as such represent approximately 5 percent of human body weight. The morphology of the calcium phosphate crystals and their characteristics were extensively studied and are widely used. For instance, phosphate and carbonate salts find use in the field of medicine, such as in the field of bone reconstruction, especially as these salts were found to be biocompatible. Similarly, also other salts like magnesium carbonate, magnesium phosphate, barium phosphate or zinc phosphate are well tolerated in the human body. Calcium carbonate (CaCO₃), calcium phosphate (Ca(H₂PO₄)₂), tricalcium phosphate (Ca₃(PO₄)₂) and hydroxyapatite (Ca₅(PO₄)₃OH) were used to coat titanium alloys in order to enhance bone-bonding when used in orthopedic surgery. Different techniques were developed to apply inorganic salts on the implants.

Salts such as inorganic salts are normally not used alone in order to coat particles comprising pharmaceutically active ingredients (API). The reason is that plasma-spraying technique that is used to deposit hydroxyapatite coating on the titanium substrate demands extremely high processing temperatures and line-of-sight application. Other techniques such as electrophoresis or cathode polarization techniques fail to completely cover the particle substrate and, on top, require sophisticated apparatus to practice the technique. Ink-jet technology that is also used in the field of implants does not enable small particle coating with sufficient yield. Barrere et al. describe a biomimetic process, which consists of soaking titanium plates under mild temperature conditions into concentrated simulated body fluid solution that had the same inorganic content as human blood plasma multiplied by five, while the pH of the solution is progressively increased (Biomaterials 23, 1921-1930, 2002).

Coating in pharmacy is regularly applied for various purposes such as for covering up the bad taste of an API or a dosage form, for controlling the release of the API, for wetting the dosage form or for protecting the API or the dosage form from the environment or vice versa. To date, calcium carbonate was mostly used in the pharmaceutical technology as a diluent or as a carrier on which API was adsorbed. Also, from the publication of Itokazu et al in the Journal of Biomedical Materials Research (39, 536-538, 1998), methotrexate has been known to be incorporated in the porous blocks of hydroxyapatite or tricalcium phosphate.

Ueno et al. describe the fabrication of nano-CaCO₃ particles with incorporated API (Journal of Controlled Release (103, 93-98, 2005)). The process described in the publication employes dissolved API, which gets incorporated in the nano-CaCO₃ particle simultaneously with the forming of the particle from the precipitated CaCO₃. The process makes use of coprecipitation.

However, despite the above-described processes there is still a need for an improved process for the manufacture of a pharmaceutical composition, preferably for an improved process for the coating of an API, particularly of a particulate API. In particular, there is a need for a process for the manufacture of a pharmaceutical composition, exhibiting improved robustness, reproducibility, control over process parameters, applicability and use. Furthermore, there is still a need for such a method being applicable also on industrial scale.

### Summary of the invention

In a first aspect, the present invention relates to a process for coating a particle comprising a pharmaceutically active ingredient (API) comprising the steps of:
a) providing a composition comprising carbonate ions, phosphate ions, or a mixture thereof,
b) providing a particle comprising an API, and
c) precipitating a carbonate salt, a phosphate salt or a mixture thereof onto the particles comprising the API, wherein the precipitation is carried out in a suspension having a pH of 4 or higher. Preferably, precipitation is carried out by providing counter ions capable of forming salts with the carbonate ions and phosphate ions from the composition of step a) under the conditions as used for precipitation. Preferably, the precipitation is carried out by combining the carbonate ions and/or the phosphate ions with the API in the suspension.

Further preferred, precipitation is carried out by providing counter ions capable of forming salts with the carbonate ions and phosphate ions from the composition of step a) under the conditions as used for precipitation. Furthermore, the composition of step a) and the particle(s) comprising an API of step b) are brought together. This bringing together can be any suitable method that is known to a person skilled in the art, such as adding, mixing, contacting, or combining.

In a second aspect, the present invention relates to a process for coating a particle comprising a pharmaceutically active ingredient (API), comprising the steps of:
a) providing a composition comprising carbonate ions, phosphate ions, or a mixture thereof,
b) providing particle(s) comprising an API, and
c) precipitating a carbonate salt, a phosphate salt or a mixture thereof onto the particle(s) comprising the API, and wherein the precipitation is achieved by gradually combining the carbonate ions, the phosphate ions or the mixture thereof with counter ions in the presence of the particles comprising the API.

Another aspect of the present invention is a particle comprising a pharmaceutically active ingredient (API), the particle being coated with a coating comprising a carbonate salt, phosphate salt, or mixture thereof.

Yet another aspect of the present invention is a pharmaceutical composition comprising one or more coated particle(s) as defined herein.

The present invention also relates to the use of the process according to the present invention, or of the coated particle according to the present invention, or of the pharmaceutical composition according to the present invention for preparing a medicament.

The present invention also relates to the use of the process according to the present invention for taste masking of a pharmaceutically active ingredient.

### Description of the invention, its advantages and preferred embodiments:

The following aspects, advantageous features and preferred embodiments of the present invention summarized in the following items, respectively alone or in combination, further contribute to solving the object of the invention:
(1) A process for coating a particle comprising a pharmaceutically active ingredient (API) comprising the steps of:
   a) providing a composition comprising carbonate ions, phosphate ions, or a mixture thereof,
   b) providing particle(s) comprising an API, and
   c) precipitating a carbonate salt, a phosphate salt or a mixture thereof onto the particles comprising the API, wherein the precipitation is carried out in a suspension having a pH of 4 or higher.

Preferably, precipitation is carried out by providing counter ions capable of forming salts with the carbonate ions and phosphate ions from the composition of step a) under the conditions as used for precipitation. Further preferred, the precipitation is carried out by combining, preferably gradually combining, the carbonate ions and/or the phosphate ions with the API in the suspension.
(2) A process for coating a particle comprising a pharmaceutically active ingredient (API), comprising the steps of:
   a) providing a composition comprising carbonate ions, phosphate ions, or a mixture thereof,
   b) providing particle(s) comprising an API, and
   c) precipitating a carbonate salt, a phosphate salt or a mixture thereof onto the particles comprising the API, and wherein the precipitation is achieved by gradually combining the carbonate ions, the phosphate ions or the mixture thereof with counter ions in the presence of the particle(s) comprising the API.
(3) The process according to items (1) or (2), wherein the gradually combining of the carbonate ions, the phosphate ions or the mixture thereof with counter ions is carried out over period of at least 1 min., preferably at least 5 min., further preferably at least 10 min., more preferably at least 30 min., and most preferably over a period of at least 60 min.
(4) The process according to any of items (1) to (3),
   wherein precipitation is achieved by combining a suspension comprising the particle(s) comprising the API and the composition of step a) with a solvent containing counter ions for the carbonate ions and/or phosphate ions, or by combining a suspension comprising the particle(s) comprising the API and counter ions for the carbonate ions and/or phosphate ions with a solvent containing the composition of step a), or by combining a suspension comprising the API with a solvent containing the composition of step a) and another solvent containing the counter ions for the carbonate ions and/or phosphate ions.
(5) The process according to any one of the preceding items, wherein the amount of the particle(s) comprising pharmaceutically active ingredient in the suspension during precipitation is in a range from 0.1 % to 50 % w/w based on the total amount of solvent(s) used in the process, preferably the amount of particle(s) is in a range from 0.1 % to 50 % w/w based on the amount of solvent(s) in the suspension during precipitation.
(6) The process according to any one of the preceding items, wherein the total amount of the carbonate ions, phosphate ions or mixture thereof is from 0.002 to 5 mol/L based on the total amount of solvent used in the process.
(7) The process according to any of items (2) to (6), wherein the total amount of counter ions for precipitating the carbonate salt(s), phosphate salt(s), or mixture thereof is from 0.002 to 5 mol/L based on the total amount of solvent used in the process.
(8) The process according to any one of the preceding items, wherein the carbonate ions, phosphate ions or mixture thereof are provided by one or more compounds selected from the group consisting of phosphorous acid, phosphorous acid water soluble salts, carbonic acid, carbonic acid water soluble salts, carbon dioxide, and mixtures thereof.
(9) The process according to the preceding item, wherein the phosphorous acid water soluble salt is selected from the group of sodium phosphate, sodium hydrogenphosphate, sodium dihydrogenphosphate, disodium diphosphate, tetrasodium diphosphate, potassium phosphate, potassium hydrogenphosphate, potassium dihydrogenphosphate, disodium pyrophosphate, tetrasodium pyrophosphate, pentapotassium triphosphate, pentasodium triphosphate, sodium polyphosphate, sodium hexametaphosphate, sodium potassium polyphosphate, Kurrol's salt (KPO₃)n, sodium tripolyphosphate, disodium phosphate, ammonium phosphate, ammonium polyphosphate, potassium polyphosphate and potassium pyrophosphate; preferably is selected from the group of sodium phosphate, sodium hydrogenphosphate, sodium dihydrogenphosphate, disodium diphosphate potassium phosphate, potassium hydrogenphosphate, potassium dihydrogenphosphate and disodium phosphate.
(10) The process according to the preceding two items, wherein the carbonic acid water soluble salt is selected from the group consisting of sodium carbonate, potassium carbonate and lithium carbonate; preferably is selected from sodium carbonate and potassium carbonate.
(11) The process according to any one of the preceding items, wherein the counter ions for precipitating a carbonate salt, phosphate salt, or mixture thereof are provided by one or more compounds selected from the group consisting of calcium salts, magnesium salts, zinc salts, barium salts, strontium salts, copper salts, iron salts and mixtures thereof; preferably from the group consisting of calcium salts, magnesium salts, zinc salts, barium salts and mixtures thereof; more preferably from the group consisting of calcium salts, magnesium salts, zinc salts and mixtures thereof.
(12) The process according to item (11), wherein the counter ions for precipitating the carbonate salt, phosphate salt, or mixture thereof are provided by one or more compounds selected from the group consisting of calcium chloride, calcium nitrate, calcium sulfate, calcium acetate, calcium citrate, magnesium chloride, magnesium nitrate, magnesium sulfate, magnesium acetate, magnesium citrate, barium chloride, barium nitrate, barium sulfate, barium acetate, strontium chloride, strontium nitrate, iron chloride, iron nitrate, iron sulfate, iron acetate, copper chloride, copper nitrate, copper sulfate, copper acetate and mixtures thereof; preferably are provided by one or more compounds selected from the group of calcium chloride, calcium nitrate, calcium acetate, magnesium chloride, magnesium nitrate, magnesium sulfate, magnesium acetate, magnesium citrate, barium chloride, barium nitrate, barium acetate and mixture thereof.
(13) The process according to any one of the preceding items, wherein the compound(s) providing the counter ions for precipitating the carbonate salt, phosphate salt, or mixture thereof has/have a solubility in an aqueous solvent of at least of about 0.002 mol/l.
(14) The process according to any one of the preceding items, wherein the pH of the suspension comprising particle(s) comprising the API during the precipitation is 4 or higher, preferably 5 or higher, more preferably 7 or higher, and most preferably 9 or higher.
(15) The process according to any one of the preceding items, wherein the pH of the suspension comprising the particle(s) comprising the API is prevented from dropping below pH 4 during the precipitation, or is prevented from getting substantially (more than 0.5 pH) lower from the starting pH during the precipitation.
(16) The process according to any one of the preceding items, wherein the pH of the solvent comprising the particle(s) comprising the API is maintained substantially (preferably not more than 1 pH deviation, further preferred not more than 0.5 pH deviation) the same during the precipitation.
(17) The process according to any one of the preceding items, wherein the suspension comprising particle(s) comprising API further comprises one or more anti-aggregating agents.
(18) The process according to any one of the preceding items, wherein the anti-aggregating agents are selected from the group consisting of surfactants, organic salts, saccharides, natural or synthetic polymers, silica derivatives, waxes and salts or mixtures thereof.
(19) The process according to any one of the previous items, further comprising a step of precipitating a compound selected from the group consisting of surfactants, organic salts, saccharides, synthetic polymers, silica derivatives, waxes and salts, or a mixture thereof, on the surface of the particle comprising an API prior to precipitating the carbonate salt, the phosphate salt or mixtures thereof.
(20) The process according to previous two items, wherein the silica derivative is silicon dioxide, silicon dioxide derivatized with an organic compound, silicon alkoxide, methanesiliconic acid sodium or methanesiliconic acid potassium.
(21) The process according to any one of previous three items, wherein the silicon dioxide derivatized with organic compound is selected from the group consisting of tetraethyl orthosilicate and bis-1,2-trietoxysilyl ethane.
(22) The process according to items (18) or (19), wherein surfactant is selected from the group consisting of nonionic surfactants, anionic surfactants, cationic surfactants and mixtures thereof, preferably is glyceryl monooleate, macrogol 15 hydroxystearate, phospholipide, polyoxyethylene sorbitan fatty acid ester, polyoxylglyceride, sorbitan fatty acid ester, lauric acid, myristyl alcohol, vitamin E polyethylene glycol succinate, perfluorooctanoate, perfluorooctanesulfonate, sodium dodecyl sulfate, ammonium lauryl sulfate, sodium laureth sulfate, alkyl benzene sulfonate, fatty acid salt, cetyl trimethylammonium bromide, hexadecyl trimethyl ammonium bromide, cetylpyridinium chloride, polyethoxylated tallow amine, benzalkonium chloride, benzethonium chloride, copolymer of poly(ethylene oxide) and poly(propylene oxide), alkyl polyglucoside, fatty alcohol, or mixture thereof.
(23) The process according to any one of items (17) to (22), wherein the anti-aggregating agent or a compound being on the surface of particle(s) comprising the API before precipitating carbonate salt, phosphate salt or mixture thereof, is acacia, agar, albumin, alginic acid, aluminum monostearate, ammonium alginate, bentonite, calcium alginate, carbomer, carboxymethylcellulose calcium, carboxymethylcellulose sodium, carrageenan, microcrystalline cellulose, carboxymethylcellulose sodium, ceratonia, ceresin, cyclodextrins, ethylene glycol stearates, glyceryl monostearate, hydroxypropyl betadex, hydroxypropyl cellulose, hypromellose, macrogol 15 hydroxystearate, magnesium aluminum silicate, pectin, pentetic acid, polyvinyl alcohol, potassium alginate, propylene glycol, propylene glycol alginate, raffinose, sodium acetate, sodium alginate, sodium borate, sorbitol, sulfobutylether β-cyclodextrin, trehalose, white wax , yellow wax , xanthan gum, zinc acetate, myristyl alcohol, ethylcellulose, gelatin, glyceryl behenate, guar gum, hydroxyethyl cellulose, hydroxyethylmethyl cellulose, hydroxypropyl starch, maltodextrin, methylcellulose, polydextrose, poly(methyl vinyl ether/maleic anhydride), saponite, sucrose, tragacanth, xanthan gum, PEG 4-100 monolaurate, PEG 4-100 monooleate, PEG 4-100 monostearate, PEG 400 distearate, PEG 100,200,300 monolaurate , PEG 100,200,300 monooleate, PEG 400 dioleate, PEG 400-1000 monostearate, PEG-1 stearate, PEG-2 stearate, PEG-2 oleate, PEG-4 laurate, PEG-4 oleate, PEG-4 stearate, PEG-5 stearate, PEG-5 oleate, PEG-6 oleate, PEG-7 oleate, PEG-6 laurate, PEG-7 laurate, PEG-6 stearate, PEG-8 laurate, PEG-8 oleate, PEG-8 stearate, PEG-9 oleate, PEG-9 stearate, PEG-10 laurate, PEG-10 oleate, PEG-10 stearate, PEG-12 laurate, PEG-12 oleate, PEG-12 ricinoleate, PEG-12 stearate, PEG-15 stearate, PEG-15 oleate, PEG-20 laurate, PEG-20 oleate, PEG-20 stearate, PEG-25 stearate, PEG-32 laurate, PEG-32 oleate, PEG-32 stearate, PEG-30 stearate, PEG-40 laurate, PEG-40 oleate, PEG-40 stearate, PEG-45 stearate, PEG-50 stearate, PEG-55 stearate, PEG-100 oleate, PEG-100 stearate, PEG-200 oleate, PEG-400 oleate, PEG-600 oleate, PEG-4 dilaurate, PEG-4 dioleate, PEG-4 distearate, PEG-6 dilaurate, PEG-6 dioleate, PEG-6 distearate, PEG-8 dilaurate , PEG-8 dioleate , PEG-8 distearate, PEG-10 dipalmitate, PEG-12 dilaurate, PEG-12 distearate, PEG-12 dioleate, PEG-20 dilaurate, PEG-20 dioleate, PEG-20 distearate, PEG-32 dilaurate, PEG-32 dioleate, PEG-32 distearate, PEG-400 dioleate, PEG-400 distearate, PEG 4-150 mono dilaurate, PEG 4-150 mono dioleate, PEG 4-150 mono distearate, PEG-20 glyceryl laurate, PEG-30 glyceryl laurate, PEG-15 glyceryl laurate, PEG-40 glyceryl laurate, PEG-20 glyceryl stearate, PEG-20 glyceryl oleate, PEG-30 glyceryl oleate, PEG-3 castor oil, PEG-5, 9, and 16 castor oil, PEG-20 castor oil , PEG-23 castor oil, PEG-30 castor oil, PEG-35 castor oil, PEG-38 castor oil, PEG-40 castor oil, PEG-50 castor oil, PEG-56 castor oil, PEG-60 castor oil, PEG-100 castor oil, PEG-200 castor oil, PEG-5 hydrogenated castor oil, PEG-7 hydrogenated castor oil, PEG-10 hydrogenated castor oil, PEG-20 hydrogenated castor oil, PEG-25 hydrogenated castor oil, PEG-30 hydrogenated castor oil, PEG-40 hydrogenated castor oil, PEG-45 hydrogenated castor oil, PEG-50 hydrogenated castor oil, PEG-60 hydrogenated castor oil, PEG-80 hydrogenated castor oil, PEG-100 hydrogenated castor oil, PEG-6 corn oil, PEG-6 almond oil, PEG-6 apricot kernel oil, PEG-6 olive oil, PEG-6 peanut oil, PEG-6 hydrogenated palm kernel oil, PEG-6 palm kernel oil, PEG-6 triolein, PEG-8 corn oil, PEG-20 corn glycerides, PEG-20 almond glycerides, PEG-25 trioleate, PEG-40 palm kernel oil, PEG-60 corn glycerides, PEG-60 almond glycerides, PEG-4 caprylic/capric triglyceride, PEG-8 caprylic/capric glycerides, PEG-6 caprylic/capric glycerides, lauroyl macrogol-32 glyceride, stearoyl macrogol glyceride, pentaerythrityl tetraisostearate, pentaerythrityl distearate, pentaerythrityl tetraoleate, pentaerythrityl tetrastearate, pentaerythrityl tetracaprylate/tetracaprate, pentaerythrityl tetraoctanoate, polyglyceryl-2 stearate, polyglyceryl-2 oleate, polyglyceryl-2 isostearate, polyglyceryl-3 oleate, polyglyceryl-4 oleate, polyglyceryl-4 stearate, polyglyceryl-6 oleate, polyglyceryl-10 laurate, polyglyceryl-10 oleate, polyglyceryl-10 stearate, polyglyceryl-6 ricinoleate, polyglyceryl-10 linoleate, polyglyceryl-6 pentaoleate, polyglyceryl-3 dioleate, polyglyceryl-3 distearate, polyglyceryl-4 pentaoleate, polyglyceryl-6 dioleate, polyglyceryl-2 dioleate, polyglyceryl-10 trioleate, polyglyceryl-10 pentaoleate, polyglyceryl-10 septaoleate, polyglyceryl-10 tetraoleate, polyglyceryl-10 decaisostearate, polyglyceryl-101 decaoleate, polyglyceryl-10 mono or dioleate, polyglyceryl polyricinoleate, propylene glycol monocaprylate, propylene glycol monolaurate, propylene glycol oleate, propylene glycol myristate, propylene glycol monostearate, propylene glycol hydroxy stearate, propylene glycol ricinoleate, propylene glycol isostearate, propylene glycol monooleate, propylene glycol dicaprylate/dicaprate, propylene glycol dioctanoate, propylene glycol caprylate/caprate, propylene glycol dilaurate, propylene glycol distearate, propylene glycol dicaprylate, propylene glycol dicaprate, oleic acid, stearic acid, monopalmitolein, monoelaidin, monocaproin, monocaprylin, monocaprin, monolaurin, glyceryl monomyristate, glyceryl monooleateglyceryl monooleate, glycerol monooleate/linoleate, glycerol monolinoleate, glyceryl ricinoleate, glyceryl monolaurate, glycerol monopalmitate, glycerol monostearate, glyceryl mono- or dioleate, glyceryl palmitic/stearic, glyceryl acetate, glyceryl laurate, glyceryl citrate/lactate/oleate/ linoleate, glyceryl caprylate, glyceryl caprylate/caprate, caprylic acid mono- or diglycerides, caprylic/capric glycerides, mono- or diacetylated monoglycerides, glyceryl monostearate, lactic acid esters of mono- or diglycerides, dicaproin, dicaprin, dioctanoin, dimyristin, dipalmitin, distearin, glyceryl dilaurate, glyceryl dioleate, glycerol esters of fatty acids , dipalmitolein, 1 ,2 and 1,3-diolein, dielaidin, dilinoleincholesterol, sitosterol, lanosterol, PEG-24 cholesterol ether, PEG-30 cholestanol, phytosterol, PEG-25 phyto sterol, PEG-5 soya sterol, PEG-10 soya sterol, PEG-20 soya sterol, PEG-30 soya sterol, PEG-10 sorbitan laurate, PEG-20 sorbitan monolaurate, PEG-4 sorbitan monolaurate, PEG-80 sorbitan monolaurate, PEG-6 sorbitan monolaurate, PEG-20 sorbitan monopalmitate, PEG-20 sorbitan monostearate, PEG-4 sorbitan monostearate, PEG-8 sorbitan monostearate, PEG-6 sorbitan monostearate, PEG-20 sorbitan tristearate, PEG-6 sorbitan tetrastearate, PEG-60 sorbitan tetrastearate, PEG-5 sorbitan monooleate, PEG-6 sorbitan monooleate, PEG-20 sorbitan monooleate, PEG-40 sorbitan oleate, PEG-20 sorbitan trioleate , PEG-6 sorbitan tetraoleate, PEG-30 sorbitan tetraoleate, PEG-40 sorbitan tetraoleate, PEG-20 sorbitan monoisostearate, PEG sorbitol hexaoleate, PEG-6 sorbitol hexastearate, PEG-2 oleyl ether, PEG-3 oleyl ether, PEG-5 oleyl ether, PEG-10 oleyl ether, PEG-20 oleyl ether, PEG-4 lauryl ether, PEG-9 lauryl ether, PEG-23 lauryl ether, PEG-2 cetyl ether, PEG-10 cetyl ether, PEG-20 cetyl ether, PEG-2 stearyl ether, PEG-10 stearyl ether, PEG-20 stearyl ether, PEG-100 stearyl ether, sucrose distearate, sucrose distearate/monostearate, sucrose dipalmitate, sucrose monostearate , sucrose monopalmitate , sucrose monolaurate, poloxamer 105, poloxamer 108, poloxamer 122, poloxamer 123, poloxamer 124, poloxamer 181, poloxamer 182, poloxamer 183, poloxamer 184, poloxamer 185, poloxamer 188, poloxamer 212, poloxamer 215, poloxamer 217, poloxamer 231, poloxamer 234, poloxamer 235, poloxamer 237, poloxamer 238, poloxamer 282, poloxamer 284, poloxamer 288, poloxamer 331, poloxamer 333, poloxamer 334, poloxamer 335, poloxamer 338, poloxamer 401, poloxamer 402, poloxamer 403, poloxamer 407, sorbitan monolaurate, sorbitan monopalmitate, sorbitan monooleate, sorbitan monostearate, sorbitan trioleate, sorbitan sesquioleate, sorbitan tristearate, sorbitan monoisostearate, sorbitan sesquistearate, ethyl oleate, isopropyl myristate, isopropyl palmitate, ethyl linoleate, isopropyl linoleate, sodium caproate, sodium caprylate, sodium caprate, sodium laurate, sodium myristate, sodium myristolate, sodium palmitate, sodium palmitoleate, sodium oleate, sodium ricinoleate, sodium linoleate, sodium linolenate, sodium stearate, sodium lauryl sulfate , sodium tetradecyl sulfate, sodium lauryl sarcosinate, sodium dioctyl sulfosuccinate, sodium cholate, sodium taurocholate, sodium glycocholate, sodium deoxycholate, sodium taurodeoxycholate, sodium glycodeoxycholate, sodium ursodeoxycholate, sodium chenodeoxycholate, sodium taurochenodeoxycholate, sodium glyco cheno deoxycholate, sodium cholylsarcosinate, sodium n-methyl taurocholate, sodium lithocholate, egg/soy lecithin lyso egg/soy lecithin, hydroxylated lecithin, lysophosphatidylcholine, cardiolipin, sphingomyelin, phosphatidylcholine, phosphatidyl ethanolamine, phosphatidic acid, phosphatidyl glycerol, phosphatidyl serine, diethanolammonium polyoxyethylene-10 oleyl ether phosphate, esterification products of fatty alcohols or fatty alcohol ethoxylates with phosphoric acid or anhydride, ether carboxylates (by oxidation of terminal OH group of fatty alcohol ethoxylates), succinylated monoglycerides, sodium stearyl fumarate, stearoyl propylene glycol hydrogen succinate, mono/diacetylated tartaric acid esters of mono- or diglycerides, citric acid esters of mono- or diglycerides, glyceryl-lacto esters of fatty lactylic esters of fatty acids, calcium/sodium stearoyl-2-lactylate, calcium/sodium stearoyl lactylate, alginate salts, propylene glycol alginate, ethoxylated alkyl sulfates, alkyl benzene sulfones, α-olefin sulfonates, acyl isethionates, acyl taurates, alkyl glyceryl ether sulfonates, octyl sulfosuccinate disodium, disodium undecylenamideo-MEA-sulfosuccinate, lauroyl carnitine, palmitoyl carnitine, myristoyl carnitine, hexadecyl triammonium bromide, decyl trimethyl ammonium bromide, cetyl trimethyl ammonium bromide, dodecyl ammonium chloride, alkyl benzyldimethylammonium salts, diisobutyl phenoxyethoxydimethyl benzylammonium salts, alkylpyridinium salts, lauryl betaine, polyoxyethylene-15 coconut amine, or any mixture or combination thereof.
(24) The process according to any one of the preceding items, wherein the pH is controlled by adding organic or mineral acid, organic or mineral base or salts thereof.
(25) The process according to any one of the preceding items, wherein the precipitation occurs while stirring the solvent at the rate of 100 to 1000 rpm.
(26) The process according to any one of the preceding items, wherein the precipitation occurs while stirring the solvent by rotating the vessel without any paddle, or while stirring the solvent with the paddle mixer.
(27) The process according to any one of the preceding items, further comprising a step of preparing a pharmaceutical composition.
(28) The process according to any of the previous item, wherein the process comprises mixing coated particle(s) comprising a pharmaceutically active ingredient with a pharmaceutically accepted excipient and/or another pharmaceutically active ingredient.
(29) The process according to any one of the two previous items, wherein the pharmaceutical composition is in the form of a dosage form.
(30) A particle comprising a pharmaceutically acrtive ingredient (API), the particle being coated with a coating comprising a carbonate salt, phosphate salt, or mixture thereof.
(31) The particle according to item (30), wherein the particle size of the particle coated with said coating is at least 0.5 µm.
(32) The particle according to item (30) or (31), wherein the coating extends over the complete surface of the particle.
(33) The particle according to item (31), wherein the coated particle additionally comprises a layer underneath said coating, wherein the layer comprises, preferably in an amount of equal to or more than 50 % w/w, at least one compound selected from the group consisting of surfactants, organic salts, saccharides, synthetic polymers, silica derivatives, waxes or salts or mixtures thereof.
(34) The particle according to the previous item, wherein the silica derivative is selected from the group consisting of silicon dioxide, silicon dioxide derivatized with an organic compound, silicon alkoxide, methanesiliconic acid sodium or methanesiliconic acid potassium.
(35) The particle according to any one of previous two items, wherein the silicon dioxide derivatized with organic compound is selected from the group consisting of tetraethyl orthosilicate and bis-1,2-trietoxysilyl ethane.
(36) The particle according to any one of previous three items, wherein the surfactant is selected from the group consisting of nonionic, anionic and cationic surfactant, preferably is glyceryl monooleate, macrogol 15 hydroxystearate, phospholipide, polyoxyethylene sorbitan fatty acid ester, polyoxylglyceride, sorbitan fatty acid ester, lauric acid, myristyl alcohol, vitamin E polyethylene glycol succinate, perfluorooctanoate, perfluorooctanesulfonate, sodium dodecyl sulfate, ammonium lauryl sulfate, sodium laureth sulfate, alkyl benzene sulfonate, fatty acid salt, cetyl trimethylammonium bromide, hexadecyl trimethyl ammonium bromide, cetylpyridinium chloride, polyethoxylated tallow amine, benzalkonium chloride, benzethonium chloride, copolymer of poly(ethylene oxide) and poly(propylene oxide), alkyl polyglucoside or fatty alcohol.
(37) A pharmaceutical composition comprising one or more particle(s) as defined in any of items (30) to (36). Preferably, the particles, which are contained in the pharmaceutical composition have a mean particle size d90 of at least 0.5 µm, more preferably every single particle contained in the pharmaceutical composition has a particle size of at least 0.5 µm.

The size of single particle in the sample can be measured by microscopy, preferably by scaning electron microscopy. Particle dimension is then determined by image analysis, either manually or automated.

A suitable collection of the particles would be characterized by measuring the volume based particle size distribution using the standard static light scattering method, where ≤10% would be smaller than 0.5 µm. A suitable method for particle dispersion prior to light scattering measurement have to be applied. The method of particle dispersion can be mixing, use of dispersant, ultrasound application or combination of these.
(38) Use of the process according to any of items (1) to (29), or of the coated particle according to items (30) to (36), or of the pharmaceutical composition according to item (37) for preparing a medicament.
(39). Use of the process according to any of items (1) to (29) for taste masking of a pharmaceutically active ingredient.
(40) Use of the process according to any one of items (1) to (29), or of the coated particle according to items (30) to (36), or of the pharmaceutical composition according to item (37) for spraying on the core, wherein the core is an inert pharmaceutically acceptable excipient or wherein the core comprises an inert pharmaceutically acceptable excipient and pharmaceutically active ingredient.

### Detailed description of the invention

While studying pharmaceutically active ingredient coating techniques it was surprisingly found that particle(s) comprising an pharmaceutically active ingredient (API) can be easily coated with a carbonate salt, phosphate salt, or a mixture thereof in a suspension, when the coating process employs precipitating the carbonate salt, phosphate salt or mixture thereof in the presence of a particle comprising API in a solvent and the pH of the solvent is 4 or higher. The process allows preparing coated particles with a high load of the API while the obtained particles still display benefits from the coating like the ability to mask taste, modify dissolution, provide mechanical protection of the particles, improve wettability, or improve bulk properties of the coated particles such as flowability, compressibility or tap density.

Alternatively, an unexpectedly uniform and sufficiently thick coating of the carbonate salt, phosphate salt, or mixture thereof was obtained on a particle comprising an API when the precipitation of the carbonate salt, phosphate salt or mixture thereof in the presence of a particle comprising API in a suspension occurred only gradually, which was achieved by continuously adding controlled portions of the carbonate ions, phosphate ions or a mixture thereof with the respective suitable counter ion for precipitating the carbonate salt, phosphate salt, or mixture thereof, over period of at least 1 minute. Improved results were obtained when the suspension was stirred during gradual addition of salts. In a further preferred embodiment, the gradual combining of the respective ions with the respective suitable counterions can be carried out over a time period of at least 5 min., preferably at least 10 min., more preferably at least 30 min. and most preferably over a time period of at least 60 min. "Gradually combining" herein denotes adding the total amount of compound, i.e. salt, used in the coating process continuously or in portions over at least 1 minute.

An important advantage of the present invention is the possibility to simultaneously coat particles having an extremely heterogeneous particle size distribution, whereas in general the coating of such particles by using different techniques such as spray techniques leads to problems. For example, micrometer size particles having a size in a range of about 0.5-400 µm can be coated together with the pellet size particles having a size in the range of about 0.5-3 mm in a single run, which allows for an improved coating process e.g. with regard to time and costs. Particularly, the absolute size of the particles that can be adequately coated with the present process can vary from a few tens of nanometer to several millimeters.

The particle(s) comprising the API used for the process according to the present invention preferably has/have a particle size of at least 0.5 µm, more preferably of at least 10 µm, most preferably in a range of from 1 µm to 1 mm. Within the meaning of the present invention, the term "particle comprising the API" denotes the particle without the coating comprising a carbonate salt, a phosphate salt or a mixture thereof precipitated according to the process of the present invention. In case the particle comprising the API is coated with the coating comprising a carbonate salt, a phosphate salt or a mixture thereof precipitated according to the process of the present invention, it is denoted as "coated particle".

According to the invention, the coated particle comprises the API in the particle, but can e.g. also comprise API in the coating comprising a carbonate salt, a phosphate salt or a mixture thereof precipitated according to the process of the present invention, or in a further coating which is applied onto the coating comprising a carbonate salt, a phosphate salt or a mixture thereof. The term "coating comprising a carbonate salt, a phosphate salt or a mixture thereof" implies that the coated particle consists at least of a particle and a coating comprising a carbonate salt, a phosphate salt or a mixture thereof, wherein the composition of the particle is distinct to the composition of the coating by means of components and/or amounts of components. Preferably, the amount of API in the coating comprising a carbonate salt, a phosphate salt or a mixture thereof precipitated according to the process of the present invention is equal to or less than 30 %, preferably equal to or less than 20 %, more preferably equal to or less than 10 %, and most preferred less than 5 %, 4 %, 3 %, 2 % or 1 % of the total amount of API in the particle comprising the API.

The coated particles comprising the API preferably have a particle size in a range of from 0.5 to 3 mm.

According to the invention, any API can be used which can be formulated into particles, optionally by using suitable excipients. According to the present invention, the particles are coated in a suspension, which means that the particles cannot be dissolved in the solvent(s) which is/are used in the process at all or are only dissolved in small amounts, preferably less than 30%, preferably, less than 20%, further preferred less than 10%, 5% or 1% of the total amount of particles is dissolved in the solvent. A possibility to avoid the dissolution of the particles in the solvent is to provide a protective coating onto the particles before carrying out the process according to the invention. Furthermore, a person skilled in the art can easily choose suitable conditions, e.g. solvent, pH or temperature, which provide for the desired low dissolution of the particles. Suitable solvents are described below.

The term "particle comprising API" and "particle(s)" as used herein refers to particles, powders, granulate, microcapsules, microspheres, pellets or the like, which can be freely flowing or agglomerated and which can be in amorphous or crystalline form or in any combination thereof. The term "particle comprising API" and "particle(s)" refers to the pharmaceutically active ingredient(s) alone (if the API in particulate form is used in the process according to the invention in pure form) or the API together with at least one further pharmaceutically active ingredient and/or at least one pharmaceutically acceptable excipient.

The pharmaceutically active ingredient can be hydrophilic, lipophilic, amphiphilic or hydrophobic, and preferably has a solubility of less than 1 mg/ml at pH 6.8 in the solvent(s) used in the process. Generally, solubility as meant herein can be measured using USP apparatus 2 with agitation at 75 RPM or the traditional shake-flask method, preferably the latter, where dissolution medium can be a phosphate buffer having the pH of 6.8 and the temperature is maintained at 37 °C. However, the best criterion to define solubility is when the pharmaceutically active ingredient has the solubility of less than 1 mg/ml in (a) solvent(s) of the process at the working temperature. The given solubility refers to the appropriate solubility that the particles comprising the API have, meaning that in the event that the API alone does not exhibit such low solubility, this can be overcome by mixing it with hydrophobic excipients, coating it with a suitable coating or undertaking any other preparatory technique to form a particle comprising the API wherein the particle has a solubility of less than 1 mg/ml at the working pH, as in this case the particles in a solvent, even though comprising the API and other excipients, possible even comprising another API, present depositing surface that is completely in line with demands and purpose of the present invention. Particles comprising an API can be adequately coated even when displaying a solubility of less than 1 mg/ml at the pH of about 4 measured at 25 °C, which would be normal operating temperature when employing the process of the present invention. In a particular aspect, the API alone has a solubility of less than 1 mg/ml at the pH of about 4 and measured at 25 °C, and preferably exhibits solubility of less than 1 mg/ml at any pH over about 4, specifically around 6.8.

Pharmaceutically active ingredient can be any agent having therapeutic or other value when administered to a mammal, particularly to an animal or human being, such as drugs or vitamins. Specific non-limiting examples of APIs that can be used in the process or later on in the pharmaceutical composition or dosage forms or for the use of the present invention include analgesics and antiinflammatory agents, anthelmintics, anti-arrhythmic agents, antiasthma agents, antibacterial agents, anti-viral agents, anti-coagulants, anti-depressants, antidiabetics, antiepileptics, anti-fungal agents, anti-gout agents, anti-hypertensive agents, anti-malarials, anti-migraine agents, anti-muscarinic agents, antineoplastic agents and immunosuppressants, anti-protozoal agents, anti-thyroid agents, anti-tussives, anxiolytic, sedatives, hypnotics and neuroleptics, β-blockers, cardiac inotropic agents, corticosteroids, diuretics, anti-parkinsonian agents, gastro-intestinal agents, histamine-receptor antagonists, keratolytics, lipid regulating agents, muscle relaxants, anti-anginal agents, nutritional agents, analgesics, sex hormones, stimulants, peptides, peptidomimetics, DNA, RNA, oligodeoxynucleotides, genetic material, proteins, oligonucleotides, and vaccines.

The pharmaceutically active ingredient can be a low molecular weight molecule of a molecular weight of less than about 1000 g/mol; or a protein, peptide, oligonucleotide, DNA or RNA, wherein molecular weight can span to more thousands g/mol. Without any limitations, examples of the pharmaceutically active ingredients include the following representative compounds, as well as their pharmaceutically acceptable salts, stereoisomers, esters or ethers: abacavir, acarbose, acebutolol, acetazolamide, acetohexamide, acrivastine, acutretin, acyclovir, alatrofloxacin, albendazole, albuterol, alclofenac, alendronate, allopurinol, aloxiprin, alprazolam, alprenolol, alprostadil, amantadine, amiloride, aminoglutethimide, amiodarone, amiodarone, amitriptyline, amlodipine, amodiaquine, amoxapine, amoxapine, amphetamine, amphotericin, amprenavir, amrinone, amsacrine, amyl nitrate, amylobarbital, amylobarbitone, aspirin, astemizole, atenolol, atorvastatin, atovaquone, atropine, auranofin, azapropazone, azathioprine, azelastine, azithromycin, baclofen, barbital, barbitone, becaplermin, beclamide, beclomethasone, bendrofluazide, benethamine, benethamine penicillin, benezepril, benidipine, benorylate, bentazepam, benzhexol, benzhexol, benznidazole, benzonatate, benztropine, bephenium hydroxynaphthoate, betamethasone, bezafibrate, bicalutamide, biperiden, bisacodyl, bisanthrene, bortezomib, bovine growth hormone, bromazepam, bromfenac, bromocriptine, bromocriptine mesylate, bromperidol, brompheniramine, brotizolam, budesonide, bumetanide, bupropion, busulphan, butenafine, butenafine, butobarbital, butobarbitone, butoconazole, butoconazole nitrate, calcifediol, calcipotriol, calciprotiene, calcitonin, calcitriol, cambendazole, camptothecan, camptothecin, candesartan, capecitabine, capsacin, capsaicin, captopril, carbamazepine, carbimazole, carbinoxamine, carbromal, carotenes, caspofungin, cefazolin, cefoxitin sodium, celecoxib, cephadrine, cephalexin, cerivistatin, cetrizine, chlopheniramine, chlophenisamine, chloproguanil, chlorambucil, chlordiazepoxide, chlormethiazole, chloroquine, chlorothiazide, chlorproguanil, chlorpromazine, chlorpropamide, chlorprothiocene, chlorprothixene, chlorthalidone, cholecalciferol, cilostazol, cimetidine, cinnarizine, cinoxacin, ciprofloxacin, ciprofloxacin, cisapride, citalopram, citrizine, clarithromycin, clemastine, clemastine fumarate, clemizole, clenbuterol, clinofibrate, clioquinol, clobazam, clofazimine, clofibrate, clomiphene, clomiphene citrate, clomipramine, clonazepam, clopidrogel, clotiazepam, clotrimazole, cloxacillin, clozapine, codeine, conjugated estrogen, cortisone acetate, cortisone acetate, cromalyn sodium, cromoglicate, cromolyn, cyclizine, cyclosporin, cyproheptadine, cyproheptadine, dacarbazine, danazol, dantrolene, dantrolene sodium, darodipine, decoquinate, delavirdine, demeclocycline, desoxymethasone, dexamphetamine, dexchlopheniramine, dexfenfluramine, dextropropyoxyphene, diamorphine, diazepam, diazoxide, dichlorophen, diclofenac, dicloxacillin, dicoumarol, dicumarol, didanosine, diethylpropion, diflunisal, digitoxin, digoxin, dihydro epiandrosterone, dihydrocodeine, dihydroergotamine, dihydroergotamine mesylate, dihydrotachysterol, diiodohydroxyquinoline, dilitazem, dilitazem, diloxanide furoate, dimenhydrinate, dinitolmide, diphenhydramine, diphenooxylate, diphenoxylate, diphenylimidazole, diphenylpyrallin, dipyridamole, dirithromycin, disopyramide, divalproen, docusate, dolasetron, domperidone, donepezil, donepezil, doxazosin, doxazosin, doxycycline, dronabinol, droperidol, dutasteride, econazole, econazole nitrate, editronate, efavirenz, elanapril, ellipticine, enalapril, enkephalin, enoxacin, enoximone, enrofloxacin, epalrestate, eperisone, ephedrine, epoprostenol, eprosartan, ergocalciferol, ergotamine, ergotamine tartrate, erythromycin, erythropoietin, essential fatty acids, estramustine, ethacrynic acid, ethambutol, ethinamate, ethinyloestradiol, ethionamide, ethopropazine, ethopropazine, ethotoin, etodolac, etoperidone, etoposide, etretinate, famcyclovir, famotidine, felbamate, felodipine, fenbendazole, fenbufen, fenfluramine, fenofibrate, fenolclopam, fenoldopam, fenoprofen, fenoprofen calcium, fentanyl, fexofenadine, finasteride, flecainide, flecainide, florfenicol, acetate, fluconazole, flucortolone, flucytosine, fludrocortisone, fludrocortisone acetate, fluexetine, flunanisone, flunarizine, flunarizine, flunisolide, flunitrazepam, fluopromazine, fluoxetine, fluoxymisterone, flupenthixol decanoate, flupentixol, flupentixol decanoate, fluphenazine, fluphenazine decanoate, flurazepam, flurbiprofen, flurithromycin, fluticasone propionate, fluvastatin, foscarnet sodium, fosinopril, fosphenytoin, fosphenytoin sodium, frovatriptan, frusemide, fumagillin, furazolidone, furosemide, furzolidone, gabapentin, gancyclovir, gemfibrozil, gentamycin, glibenclamide, gliclazide, glipizide, glucagon, glybenclamide, glyburide, glyceryl trinitrate, glymepiride, glymepride, granisetron, granulocyte stimulating factor, grepafloxacin, griseofulvin, guanabenz, guanabenz acetate, halofantrine, halofantrine, haloperidol, hydrocortisone, hyoscyamine, ibufenac, ibuprofen, imipenem, indinavir, indivir, indomethacin, insulin, interleukin-3, irbesartan, irinotecan, isosorbide dinitrate, isosorbide mononitrate, isotretinoin, isoxazole, isradipine, itraconazole, ivermectin, ketoconazole, ketoprofen, ketorolac, ketotifen, labetalol, lamivudine, lamotrigine, lanatoside C, lanosprazole, leflunomide, levofloxacin, levothyroxine, lisinopril, lomefloxacin, lomustine, loperamide, loratadine, lorazepam, lorefloxacin, lormetazepam, losartan, lovastatin, L-thryroxine, lysuride, lysuride maleate, maprotiline, maprotiline, mazindol, mebendazole, meclofenamic acid, meclozine, meclozine, medazepam, medigoxin, medroxyprogesterone acetate, mefenamic acid, mefloquine, mefloquine, megesterol acetate, melonicam, melphalan, mepacrine, mepenzolate bromide, meprobamate, meptazinol, mercaptopurine, mesalazine, mesoridazine, mesoridiazine, mestranol, mesylate, metformin, methadone, methaqualone, methoin, methotrexate, methoxsalen, methsuximide, methylphenidate, methylphenobarbital, methylphenobarbitone, methylprednisolone, methyltestosterone, methysergide, methysergide maleate, metoclopramide, metolazone, metoprolol, metronidazole, mianserin, mianserin, micofenolat mofetil, miconazole, midazolam, miglitol, minoxidil, misoprostenol, mitomycins, mitotane, mitoxantrone, mofetil, molindone, montelukast, morphine, mortriptyline, moxifloxacin, moxifloxacin, mycophenolate, nabumetone, nadolol, nalbuphine, nalidixic acid, naproxen, naratriptan, naratriptan, natamycin, nedocromil sodium, nefazodone, nelfinavir, nerteporfin, neutontin, nevirapine, nicardipine, nicardipine, nicotine, nicoumalone, nifedipine, nilutamide, nimesulide, nimodipine, nimorazole, nisoldipine, nitrazepam, nitrofurantoin, nitrofurazone, nizatidine, non-essential fatty acids, norethisterone, norfloxacin, norgestrel, nortriptyline, nystatin, oestradiol, ofloxacin, olanzapine, olmesartan, omeprazole, ondansetron, ondansetron, oprelvekin, omidazole, oxacillin, oxamniquine, oxantel, oxantel embonate, oxaprozin, oxatomide, oxazepam, oxcarbazepine, oxfendazole, oxiconazole, oxprenolol, oxybutynin, oxyphenbutazone, oxyphencylcimine, oxyphencylcimine, paclitaxel, pamidronate, paramethadione, paricalcitol, paroxetine, paroxetine, penicillins, pentaerythritol tetranitrate, pentazocine, pentobarbital, pentobarbitone, pentoxifylline, perchloperazine, perfloxacin, pericyclovir, perphenazine, perphenazine pimozide, phenacemide, phenbenzamine, phenindione, pheniramine, phenobarbital, phenobarbitone, phenoxybenzamine, phenoxybenzamine, phensuximide, phentermine, phenylalanine, phenylbutazone, phenytoin, physostigmine, phytonodione, pimobendan, pimozide, pindolol, pioglitazone, piroxicam, pizotifen, pizotifen maleate, pramipexol, pramipexole, pranlukast, pravastatin, praziquantel, prazosin, prazosin, prednisolone, prednisone, pregabalin, primidone, probenecid, probucol, procarbazine, procarbazine, prochlorperazine, progesterone, proguanil, proguanil, propofol, propranolol, propylthiouracil, pseudoephedrine, pyrantel, pyrantel embonate, pyridostigmine, pyrimethamine, quetiapine, quinapril, quinidine, quinidine sulfate, quinine, quinine sulfate, rabeprazole, rabeprazole sodium, raloxifene, raloxifene, ranitidine, ranitidine, recombinant human growth hormone, refocoxib, remifentanil, repaglinide, reserpine, residronate, retinoids, ricobendazole, rifabutin, rifabutine, rifampicin, rifampin, rifapentine, rimantadine, rimexolone, risperodone, ritonavir, rizatriptan, rizatriptan benzoate, robinirole, romifentanil, ropinirole, rosiglitazone, rosuvastatine, roxatidine, roxithromycin, salbutamol, salmon calcitonin, saquinavir, selegiline, sertindole, sertraline, sertraline, sibutramine, sibutramine, sildenafil, sildenafil citrate, simvastatin, sirolimus, sodium cefazoline, somatostatin, sparfloxacin, spiramycins, spironolactone, stanozolol, stavudine, stavueline, stiboestrol, sulconazole, sulconazole nitrate, sulfabenzamide, sulfacetamide, sulfadiazine, sulfadoxine, sulfafurazole, sulfamerazine, sulfamethoxazole, sulfapyridine, sulfasalazine, sulindac, sulphabenzamide, sulphacetamide, sulphadiazine, sulphadoxine, sulphafurazole, sulphamerazine, sulphamethoxazole, sulphapyridine, sulphasalazine, sulphin-pyrazone, sulpiride, sulthiame, sumatriptan, sumatriptan, succinate, tacrine, tacrolimus, tamoxifen, tamoxifen citrate, tamsulosin, tamsulosin, targretin, tazarotene, telmisartan, temazepam, teniposide, terazosin, terazosin, terbinafine, terbutaline, terbutaline sulfate, terconazole, terenadine, terfenadine, testolactone, testosterone, tetracycline, tetrahydrocannabinol, tetramisole, thiabendazole, thioguanine, thioridazine, tiagabine, tiagabine, tibolone, ticlidopine, ticlopidine, tiludronate, timolol, tinidazole, tioconazole, tirofibran, tizanidine, tizanidine, tolazamide, tolbutamide, tolcapone, tolmetin, tolterodine, topiramate, topotecan, topotecan, toremifene, toremifene citrate, tramadol, trazodone, trazodone, tretinoin, triamcinolone, triamterene, triazolam, trifluoperazine, trimetazidine, trimethoprim, trimipramine, trimipramine maleate, troglitazone, tromethamine, tropicamide, trovafloxacin, tumor necrosisi factor, undecenoic acid, ursodeoxycholic acid, valacylcovir, valproic acid, valsartan, vancomycin, vasopressin, venlafaxine, verteporfin, vigabatrin, vinblastine, vincristine, vinorelbine, vitamin A, vitamin B2, vitamin D, vitamin E and vitamin K, vitamin K5, vitamin K6, vitamin K7, vitamin K-S (II), zafirlukast, zileuton, zolmitriptan, zolpidem, and zopiclone. If desired, salts, metabolic precursors, derivatives and mixtures of pharmaceutically active ingredients may also be used. Suitable for coating is any API that has low solubility in medium where the coating takes place. In addition, the composition and pH of a medium can be selected to suit best for coating of the specific API.

Particles which can be used in the process according to the invention can be prepared by any commonly known technique. For example, particles containing one or more APIs can be prepared by using spray-drying or granulating techniques. Suitable excipients which provide the required properties for the respective preparation method to be used are well known in the art. However, it is also possible to use the API in the pure form.

The term "coated particle" as used herein refers to a coated particle which is prepared by the process according to the present invention. Thus, a coated particle comprises at least the particle comprising at least one API, wherein this particle is coated with a coating comprising carbonate and/or phosphate salts. The coated particle according to the present invention can furthermore comprise additional coating layers and excipients as described herein. Within the context of the present invention, the composition of the coating comprising the carbonate salt and/or phosphate salt preferably is different to the composition of the particle comprising the API which is coated with the coating comprising the carbonate salt and/or phosphate salt. By definition, a coating, i.e. its composition or structure like crystal structure, is different to the material which is directly beneath (underneath) the coating.

According to the present invention, the precipitation of the carbonate salt(s), phosphate salt(s) or mixtures thereof takes place in a suspension of API in a solvent or a mixture of solvents. However, it is also possible to coprecipitate further salts than the aforementioned. The term "precipitation" as used within the context of the present invention refers to the part of the process wherein the carbonate ions and/or phosphate ions and optionally further ions are brought together with the counter ions in the same solvent in the presence of the API.

The term "counter ions" as used within the context of the present invention refers to organic or inorganic ions which form salts with the carbonate ions and/or phosphate ions and optionally further ions on the surface of the API under the conditions used in the process.

The precipitation can be carried out in various ways. As mentioned above, the aim is to bring together carbonate ions, phosphate ions, or a mixture thereof and counter ions for precipitating the carbonate salts, phosphate salts, or mixture thereof in the presence of the particle comprising the API in a solvent, wherein the requirement of the pH or gradual mixing is met. When the carbonat ions and/or phosphate ions and optional further ions and the counter ions are brought together in the solvent and there are particles present, the salt precipitates and starts to overlay the particle.

One possibility of applying the process is to provide a suspension containing the particles comprising the API(s) and the carbonate ions and/or phosphate ions and optional further ions. In this embodiment of the present invention, a suspension comprising the particles comprising API can be provided firstly, which is then supplemented with a solvent comprising the carbonate ions, phosphate ions, or mixture thereof which are dissolved in the solvent. Alternatively, the carbonate ions and phosphate ions and optional further ions can be directly added in pure form, e.g. as salts, into the suspension containing the particles. Of course, the particles comprising the API(s) can also be added to a solution of the carbonate ions and/or phosphate ions or to the solid salts comprising the carbonate ions and/or phosphate ions, whereupon the solvent is added to provide the desired suspension. The counter ions for precipitating the carbonate salt, phosphate salt, or mixture thereof can also be provided in a solvent in suspended or dissolved form, preferably in dissolved form and added to the suspension containing particles comprising the API and the aforementioned ions. Alternatively the counter ions can also be added as salt(s), which, however, is less preferred as this will lead to inhomogeneous conditions during the precipitation, which leads to non-uniformly coated particles.

In this embodiment, the solvent comprising the carbonate ions and/or phosphate ions and optional further ions or the pure form of the carbonate ions and/or phosphate ions and optional further ions as well as the solvent comprising the counter ions or the counter ions in pure can be added to the suspension comprising the particles in any order, either simultaneously or sequentially. This means that it is possible to add the total amount of the carbonate ions and/or phosphate ions and optional further ions to the suspension containing the particles, but it is also possible to firstly add only a part of the carbonate ions and/or phosphate ions and optional further ions and then add the total amount or a part of the amount of the counter ions to the suspension. It is also possible to start with the addition of the counter ions to the suspension. It is also possible to add the the carbonate ions and/or phosphate ions and optional further ions and the counter ions simultaneously. However, it is less preferred to firstly combine the carbonate ions and/or phosphate ions and optional further ions with the counter ions in absence of the particles containing the API(s). However, it is possible to combine all ions firstly under conditions where no precipitation occurs and than add the particles containing the API(s). Subsequently, the conditions can be adjusted to provide precipitation of the desired salts, e.g. by increasen the pH of the suspension to a value where precipitation occurs.

In a further embodiment of the present invention, a suspension of particles comprising the API(s) and counter ions are provided in a solvent. Firstly, the counter ions for precipitating the carbonate salt(s), phosphate salt(s), or mixture thereof can be provided in a solvent, preferably as a solution, and can be added to the suspension containing the particles comprising the API(s). As described above, it is also possible to combine the particles with the counter ions as salts also in a solid state and subsequently solvent(s) can be added to provide the desired suspension and the suspension can then be supplemented with the carbonate ions, phosphate ions, or mixture thereof in a separate solvent. Less preferred, the carbonate ions and/or phosphate ions and optional further ions can be directly added to the suspension in their pure form, e.g. as salts.

Additional variations of the process will be apparent to a person skilled in the art. The solvent used herein is preferably water, which can be optionally supplemented with organic solvent(s) such as for example acetone, ethanol, isopropanol or the like up to 10 volume %. Preferably the solvent consists substantially of water, i.e. at least 95% water, more preferably the solvent is only water. Supplementing water with organic solvent influences the solubility of the salts comprising the carbonate ions, phosphate ions, optional further ions and counter ions in the respective solvents/suspension containing the API(s). Also, minute amounts of organic solvents in the suspension, where precipitation occurs can advantageously prevent that carbonate or phosphate salts, which are precipitated but not precipitated on the surface of the particles comprising the API(s) are deposited on the surface of the particle but remain free floating in the solvent. The particles which are deposited but which do not contain particles comprising the API(s) can then be separated from the desired coated particles.

The amount of particles comprising pharmaceutically active ingredient in the solvent should be choosen in order that the suspension has desired physical properties, e.g. a suitable viscosity, to carry out the precipitation. Although the suspension containing the particles and further ions/counter ions as described above will be diluted when adding the remaining ions/counter in dissolved form, it is preferred that the suspension has a specific amount of particles at the beginning of the precipitation and during the precipition.

In general, it is preferred that the amount of particles comprising the API(s) is 0.1 % to 50 % w/w, preferably of 0.1 % to 30 % w/w, more preferably of 0.1 % to 20 % w/w, yet more preferably of 0.1 % to 10 % w/w based on the total amount of solvent(s) used in the process. Further preferred, the amount of particles comprising the API(s) in the suspension during the precipitation is 0.1 % to 50 % w/w, preferably of 0.1 % to 30 % w/w, more preferably of 0.1 % to 20 % w/w, yet more preferably of 0.1 % to 10 % w/w based on the amount of solvent(s) in the suspension during precipitation. This means that during the whole process of precipitation, the suspension comprising the particles comprising the API contains the solvent in an amount to ensure that the suspension has advantageous properties, e.g. with respect to viscosity. This means that when precipitation is started by e.g. adding the first portion of respective ions/counter ions in a solvent, the suspension can be stirred in a manner suitable to provide a uniform coating on the particles by e.g. avoiding high local concentrations of ions and counter ions. On the other hand, the suspension should also have suitable properties at the end of the precipitation step when the ions and counter ions have been combined. This means that the amount of solvent which is added during the precipitation step should not be such high that the minimum amount of particles comprising the API in the suspension gets below the specified value. Otherwise, the concentration of ions and counter ions would get rather low which can lead to a rather slow and incomplete precipitation of the ions and counter ions. It is meant herein that the amount of particles refers to the amount of particles comprising the API(s) and any further excipients or optional further coatings. As mentioned above, the amount of particles in the suspension affects the handling properties of the suspension. With particle mass constituting for up to 50 % of suspension mass it is still possible to achieve suspensions displaying advantageous properties with respect to flowability and viscosity properties.

The concentration of the carbonate ions, phosphate ions or mixture thereof and optional further ions is choosen to provide the best precipitation conditions. Preferably homogenous precipitation conditions are provided, which means that concentration gradients should be avoided. This can be achieved by providing a sufficient stirring rate and/or providing the respective suspension/solutions with suitable concentrations of ions/counter ions. As described above, various methods for combining the particles comprising the API(s) with the respective ions exist. Of course, the concentration of ions in the suspension containing the particles will vary during the process as the amount of solvent varies due to the addition of further solvent(s) containing the ions/counter ions. However, the concentration of ions/counter ions also varies due to the precipitation of the respective salts.

In general, the concentration of the total amount of carbonate ions, phosphate ions or mixtures thereof is preferably from 0.002 to 5 mol/I based on the total amount of solvent which is used in the process. It is understood that the aforementioned concentration is only a theoretical concentration which can only be calculated but not measured during the process. The reason for this is that at the point of time during the process, where all ingredients have been combined, precipitation already starts and the concentration of ions/counter ions is decreased. Preferably the concentration can be from 0.025 to 5 mol/l, further preferred from 0.05 to 5 mol/l, even further preferred from 0.08 to 5 mol/l, further preferred from 0.15 to 5 mol/I and most preferred from 0.3 to 5 mol/l.

A person skilled in the art will be able to choose suitable amounts of solvent(s) for e.g. dissolving salts comprising ions/counter ions and provide a suitable concentration of the solution which is added to the suspension comprising the particles and, eventually, already the respective ions/counter ions for precipitation. As discussed above, a person skilled in the art will consider the physical properties of the suspension containing the particles comprising the API(s) when choosing the amounts of solvents for the solutions and the suspension comprising the particles.

The same concentrations are applied for the counter ion for precipitating the carbonate salt(s), phosphate salt(s), or mixture thereof. Thus, the concentration of the total amount of counter ions is preferably from 0.002 to 5 mol/I based on the total amount of solvent which is used in the process. Preferably the concentration can be from 0.025 to 5 mol/l, further preferred from 0.05 to 5 mol/l, even further preferred from 0.08 to 5 mol/l, further preferred from 0.15 to 5 mol/I and most preferred from 0.3 to 5 mol/l.

Furthermore, the nature of the particles comprising the API, e.g. their functional groups, might influence the precipitation process. For example, in cases where the particles comprise the API, which contains ionized functional groups, it is beneficial to lead the process by starting first to enrich the solvent with the particles with the ions of opposite charge to the charge of the ionized functional groups of the ingredient. This allows arranging ions in a solution around the particles and causing that subsequent precipitation takes place in a direct vicinity of the particles, which increases the chances of coating formation over new nucleation site growth. In any event, the process according to the present invention advantageously enables coating of particles with carbonate salts, phosphate salts or mixtures thereof, although salts normally do not tend to stick to the particles' surface while being precipitated, let alone to deposit on the surface in quantities sufficient to cover the surface. The present process does not require the use of any sophisticated equipment but allows accurately controlling the process parameters like pH, ion or salt concentration and the rate of mixing the ions or salts. The aforementioned parameters can be easily monitored and adjusted by using simple means. The process of the present invention yields superiorly uniform coating and enables to completely cover the particles. Furthermore, the process is suitable for coating the particles of far smaller size than mostly applied methods for coating particles like the spray-drying process. In addition, the size distribution of the particles that are suitable for the process of the present invention is not as limited as in methods like dip coating or spray drying.

The source of the carbonate ions, phosphate ions or mixture thereof that get coupled with its counter ion during the process can be without limitations phosphorous acid, phosphorous acid water soluble salt, carbonic acid, carbonic acid water soluble salt, carbon dioxide, or mixture thereof. Carbon dioxide can be introduced to the solvent by bubbling the solvent with gaseous carbon dioxide. Phosphorous acid water soluble salt used in the process can be for example sodium phosphate, sodium hydrogen phosphate, sodium dihydrogenphosphate, disodium diphosphate, tetrasodium diphosphate, potassium phosphate, potassium hydrogenphosphate, potassium dihydrogenphosphate, disodium pyrophosphate, tetrasodium pyrophosphate, pentapotassium triphosphate, pentasodium triphosphate, sodium polyphosphate, sodium hexametaphosphate, sodium potassium polyphosphate, Kurrol's salt (KPO₃)n, sodium tripolyphosphate, disodium phosphate, ammonium phosphate, ammonium polyphosphate, potassium polyphosphate or potassium pyrophosphate; preferably is sodium phosphate, sodium hydrogenphosphate, sodium dihydrogenphosphate, disodium diphosphate potassium phosphate, potassium hydrogenphosphate, potassium dihydrogenphosphate and disodium phosphate. Carbonic acid water soluble salt can be sodium carbonate, potassium carbonate, lithium carbonate or the like; preferably is sodium carbonate or potassium carbonate. In principle, any carbonate or phosphate salt is suitable for the process as long as it has a solubility in an aqueous solvent at least of about 0.002 mol/l. As discussed above, the aforementioned source of carbonate ions and phosphate ions and optional further ions can be combined with the particles comprising the API(s) either in pure form or in dissolved form.

In addition, counter ions suitable for precipitating the carbonate salt(s), phosphate salt(s), or mixture have to be chosen. Counter ions are preferably selected from the group consisting of calcium, magnesium, zinc, barium, strontium, copper and iron; preferably the counter ions are selected from the group of calcium, magnesium, zinc, barium and mixture thereof; more preferably selected from the group of calcium, magnesium, zinc and mixture thereof. The counter ion are provided by the corresponding calcium salt(s), magnesium salt(s), zinc salt(s), barium salt(s), copper salt(s), iron salt(s) or mixtures thereof. The most common salts to be used as an origin for the counter ions are for example calcium chloride, calcium nitrate, calcium sulfate, calcium acetate, calcium citrate, magnesium chloride, magnesium nitrate, magnesium sulfate, magnesium acetate, magnesium citrate, barium chloride, barium nitrate, barium sulfate, barium acetate, strontium chloride, strontium nitrate, iron chloride, iron nitrate, iron sulfate, iron acetate, copper chloride, copper nitrate, copper sulfate and copper acetate. Preferably the salts like calcium chloride, calcium nitrate, calcium acetate, magnesium chloride, magnesium nitrate, magnesium sulfate, magnesium acetate, magnesium citrate, barium chloride, barium nitrate or barium acetate are used. Similarly to the requirements for carbonate or phosphate salts, the salts which serve as a source of the counter ions are suitable if their solubility in respective solvent(s) to be used in the process is at least 0.002 mol/l. In both cases, salts to be used to introduce ions or counter ions in the solvent suspension can be in a hydrated form, which can enhance their solubility in the solvent. In addition, small quantities of organic solvent can be added to facilitate solubility. After mixing with the suspension containing the particles, the solubility is reduced and precipitation promoted.

In one aspect of the present invention, a sufficiently high pH is important as it shifts the equilibrium of a precipitation reaction for carbonate salts or phosphate salts from dissolved state towards precipitated product. When the pH is at least about 4, the formation of the salt precipitate prevails over the dissolution rate of the salt. This facilitates the production of the salt coating on the surface of the particles that are present in the solvent. However, when the salt(s) start(s) to precipitate, it (they) might form new nucleation sites, which are distinct from the particles comprising pharmaceutically active ingredient already present in the suspension. As precipitation process progresses, the newly precipitated salt(s) can deposit either on the particles that were originally present in the suspension, on the salt(s) which have already been deposited on the particles or on the newly grown nucleation sites, which leads to losses and impurities. Separate nucleation sites form when the local concentration of the salt in the solvent exceeds the solubility limit of the salt in the same solvent. Therefore, according to another aspect of the present invention, the process for coating the particles comprising the API is thus advantageously carried out by using increased process duration. The aim is to introduce (gradually) only limited amounts of ions and counter ions to the suspension comprising the particles at a well controlled rate in order to prevent to significantly exceed the solubility limit of the carbon or phosphate salt(s) in the suspension.

Unexpectedly, combining the carbon or phosphate ions with its counter ions by the stepwise addition of small amounts of the ions/counter ions, preferably over a period of at least 1 minute, resolves in precipitate formation in controlled and regular fashion, predominantly on the particle surface and the already deposited salts on the particle surface. Thus, if the time during which precipition occurs in the suspension is increased and the subsequent addition of further amounts of ions/counter ions is delayed,the formation of new nucleation sites is reduced and the process directed towards coat formation on the particles, which in turn decreases the amount of grown nucleus consisting solely of salts. Accordingly, it is preferred to carry out the step of combining the ions/counter ions in the suspension comprising the particles containing the API(s) over a period of time of more than 1 minute, further preferred of more than 5 minutes, even further preferred of more than 10 minutes, further preferred of more than 20 minutes, even further preferred of more than 40 minutes and most preferred of more than 1 hour.

Also, longer precipitation times result in the formation of more uniform and more resistant coatings, whereas the process conducted in less than a minute yields coatings of only low hardness that tend to scale and flake off from the surface of the particle. The optimum time for precipitation is mainly dependent on the ion concentrations used, solvent volumes applied and mixing speed. The precipitation time is normally proportionally decreased with the increased concentration of ions and/or the volume used. For practical reasons the process would customary not be prolonged indefinitely. At first, an experiment with the API particles at issue can be performed to determine how the process behaves with said specific API. The properties of the formed coating can also be evaluated and the information can be used to adjust the precipitation time and the time which is used for combining the ions/counter ions in order to provide coatings having the desired characteristics.

In a preferred embodiment, satisfying both the requirements of the pH and gradual mixing of the carbonate ions, phosphate ions or mixtures thereof with the counter ions for precipitating the carbonate salt(s), phosphate salt(s), or mixture thereof provides that the process is highly efficient, easy to perform and provides particles having properties which are even further improved compared to the known particles. Furthermore, by controlling variables like pH and the mixing rate of the ions during the precipitation, the process can be controlled and the desired properties of the coated particles, e.g. their particle size, and the mechanical properties of the coating, can be provided. Using a higher pH during the precipitation step enhances the resistance of the coatings to mechanical stress.

The pH of the suspension, where the particle comprising API is present and precipitation occurs, is preferably at least 4, preferably at least 5, more preferably at least 7, yet more preferably at least 9. The pH of the process can be attained by adding organic or mineral acids, organic or mineral bases, or salts thereof; i.e. hydrochloric acid, methansulpfonic acid, sulfuric acid, ammonia, sodium hydroxide, potassium hydroxide, sodium hydrogen carbonate, sodium carbonate, or the like. The pH of the solvent during the precipitation process can be monitored by a pH meter using a glass electrode. Usually, a pH meter apparatus does not immediately sense minute or rapid shifts in the pH while the source of carbonate, phosphate ions or counter ions for precipitation of are added to the suspension. Thus, the aforementioned pH limits depend significantly on the error that the pH meter does in real time measurements. Thus, the above indicated minimum pH values are meant to include also values which differ by up to 10% due to measuring inaccurancy. In practice, the pH of the solvent during the process can be adjusted by using automatic pipetor connected to the pH meter, ensuring only limited errors of the pH during the process. Preferably, the pH of the suspension, with the particles comprising the pharmaceutically active ingredient, is prevented from dropping below pH 4 during the precipitation, preferably during the whole process. In another preferred embodiment, the solvent is prevented from getting substantially lower than the starting pH during the precipitation, again preferably during the whole process. In both embodiments (controlling pH and controlling the time of combining ions and counter ions), the riskto shift the equilibrium towards salt dissolution is prevented and thus the risk of getting thin, cracked, and non uniform coatings is minimized. The properties of the carbonate or phosphate salt coatings are improved if the pH of the suspension, where the particles comprising pharmaceutically active ingredient are present, is maintained substantially the same during the precipitation. Preferably the pH is kept well over 5, preferably over 8, more preferably is around 9, or can be even higher. The specified pH is in a range where the dissolution of carbonate or phosphate salts in aqueous solvent is insignificant.

It is additionally preferred that the suspension, namely the solvent with the particles (the pharmaceutically active ingredient in particulate form) comprises one more anti-aggregating agents, which stabilize the suspension and prevents spontaneous particle aggregation or sedimentation. The formation of a cake consisting of particles is especially undesired. The anti-aggregating agents that can easily be employed in the process can be for example surfactants, organic salts, saccharides, natural or synthetic polymers, silica derivatives, waxes and salts or mixture thereof. Saccharides can be any monomeric, dimeric, oligomeric or polymeric saccharides such as sucrose, modified or unmodified cellulose, alginate or starch. Natural or synthetic polymers can be any substances like albumin, tragacanth, xanthan gum, but also modified or unmodified cellulose or the like. Silica derivatives can be silicon dioxide, silicon dioxide derivatized with an organic compound, silicon alkoxide, methanesiliconic acid sodium, methanesiliconic acid potassium or the like. Alkyl moieties in the silicon alkoxides can be any linear or nonlinear C₁-C₆ alkyl. Silicon dioxide derivatized with organic compound can be without limitations for example tetraethyl orthosilicate or bis-1,2-trietoxysilyl ethane. The surfactants can be selected from the group consisting of nonionic surfactants, anionic surfactants, cationic surfactants and mixtures thereof, preferably the surfactant is glyceryl monooleate, macrogol 15 hydroxystearate, a phospholipide, polyoxyethylene sorbitan fatty acid ester, polyoxylglyceride, sorbitan fatty acid ester, lauric acid, myristyl alcohol, vitamin E polyethylene glycol succinate, perfluorooctanoate, perfluorooctanesulfonate, sodium dodecyl sulfate, ammonium lauryl sulfate, sodium laureth sulfate, alkyl benzene sulfonate, fatty acid salt, cetyl trimethylammonium bromide, hexadecyl trimethyl ammonium bromide, cetylpyridinium chloride, polyethoxylated tallow amine, benzalkonium chloride, benzethonium chloride, copolymer of poly(ethylene oxide) and poly(propylene oxide), alkyl polyglucoside, fatty alcohol, or a mixture thereof.

It is additionally preferred that the process according to the present invention further comprises depositing a compound selected from any compound that is also suitable as an anti-aggregating agent on the surface of the particles comprising the API before precipitating carbonate salt(s), phosphate salt(s) or mixture thereof. The compounds suitable for depositing prior to precipitating carbonate salt(s), phosphate salt(s) or mixtures thereof can be the same as the anti-aggregating agents as indicated above.

The aforementioned anti-aggregation compounds display anti-aggregating agent function immediately after being added to the suspension with the particles, and stabilize the suspension. They can be applied at any stage of the process. The earlier the the anti-aggregating compound(s) is (are) employed, the higher is the anti-aggregation effect during the precipitation process. However, the anti-aggregation agents can also be used to stabilize the suspension at the end of the process, even after the precipitation step has ended. The main purpose of depositing the compound on the surface of the particle prior to the precipitation of the carbonate/phosphate salts is to promote the coating formation on the surface of the particles.

It has been observed that with certain pharmaceutically active ingredients the efficiency of the process is significantly enhanced when the particle surface is primed with the abovementioned compound. This is significant for APIs that do not offer suitable nucleation site for phosphate and/or carbonate salts. Preferably the particle surface of API is primed whose surface chemistry does not sufficiently promote nucleation of the carbonate/phosphate salts. This can be done for example by soaking the particles in a solution of the anti-aggregation compound(s) and removing the solvent(s). Other methods like powder coating or spray-drying can also be applied. This step tremendously increases the efficiency of the coating process. In a preferred embodiment of the invention the layer comprising the anti-aggregation compound(s) is provided by precipitating the anti-aggregation comounds as salts onto the particles comprising the API(s) by using the method according to the invention as described above with respect to the layer comprising carbonate and/or phosphate salts. Preferably, the process for providing the aforementioned layers is carried out as a one-pot synthesis wherein firstly the anti-aggregation salts are desposited and, after the precipitation has ended, the carbonat and/or phosphate ions and further optional ions are added and precipitated onto the first layer.

It is additionally preferred to apply a further coating comprising carbonate and/or phosphate salts on the surface of the particles comprising the API(s). Such an additional coating can be applied in addition to or instead of the aforementioned coating with an anti-aggregation compound. Thus, multi layered coatings can be provided by the process according to the invention that have different properties than monolayered coatings. The properties of such multi-layered coatings depend on the compound(s) chosen for an intermediate layer(s).

The compounds which can be used as anti-aggregating agents or as compounds which can be deposited on the surface of the particles can be without limitations acacia, agar, albumin, alginic acid, aluminum monostearate, ammonium alginate, bentonite, calcium alginate, carbomer, carboxymethylcellulose calcium, carboxymethylcellulose sodium, carrageenan, microcrystalline cellulose, carboxymethylcellulose sodium, ceratonia, ceresin, cyclodextrins, ethylene glycol stearates, glyceryl monostearate, hydroxypropyl betadex, hydroxypropyl cellulose, hypromellose, macrogol 15 hydroxystearate, magnesium aluminum silicate, pectin, pentetic acid, polyvinyl alcohol, potassium alginate, propylene glycol, propylene glycol alginate, raffinose, sodium acetate, sodium alginate, sodium borate, sorbitol, sulfobutylether β-cyclodextrin, trehalose, white wax , yellow wax , xanthan gum, zinc acetate, myristyl alcohol, ethylcellulose, gelatin, glyceryl behenate, guar gum, hydroxyethyl cellulose, hydroxyethylmethyl cellulose, hydroxypropyl starch, maltodextrin, methylcellulose, polydextrose, poly(methyl vinyl ether/maleic anhydride), saponite, sucrose, tragacanth, xanthan gum, polyethylene glycol (PEG) 4-100 monolaurate, PEG 4-100 monooleate, PEG 4-100 monostearate, PEG 400 distearate, PEG 100,200,300 monolaurate , PEG 100,200,300 monooleate, PEG 400 dioleate, PEG 400-1000 monostearate, PEG-1 stearate, PEG-2 stearate, PEG-2 oleate, PEG-4 laurate, PEG-4 oleate, PEG-4 stearate, PEG-5 stearate, PEG-5 oleate, PEG-6 oleate, PEG-7 oleate, PEG-6 laurate, PEG-7 laurate, PEG-6 stearate, PEG-8 laurate, PEG-8 oleate, PEG-8 stearate, PEG-9 oleate, PEG-9 stearate, PEG-10 laurate, PEG-10 oleate, PEG-10 stearate, PEG-12 laurate, PEG-12 oleate, PEG-12 ricinoleate, PEG-12 stearate, PEG-15 stearate, PEG-15 oleate, PEG-20 laurate, PEG-20 oleate, PEG-20 stearate, PEG-25 stearate, PEG-32 laurate, PEG-32 oleate, PEG-32 stearate, PEG-30 stearate, PEG-40 laurate, PEG-40 oleate, PEG-40 stearate, PEG-45 stearate, PEG-50 stearate, PEG-55 stearate, PEG-100 oleate, PEG-100 stearate, PEG-200 oleate, PEG-400 oleate, PEG-600 oleate, PEG-4 dilaurate, PEG-4 dioleate, PEG-4 distearate, PEG-6 dilaurate, PEG-6 dioleate, PEG-6 distearate, PEG-8 dilaurate , PEG-8 dioleate , PEG-8 distearate, PEG-10 dipalmitate, PEG-12 dilaurate, PEG-12 distearate, PEG-12 dioleate, PEG-20 dilaurate, PEG-20 dioleate, PEG-20 distearate, PEG-32 dilaurate, PEG-32 dioleate, PEG-32 distearate, PEG-400 dioleate, PEG-400 distearate, PEG 4-150 mono dilaurate, PEG 4-150 mono dioleate, PEG 4-150 mono distearate, PEG-20 glyceryl laurate, PEG-30 glyceryl laurate, PEG-15 glyceryl laurate, PEG-40 glyceryl laurate, PEG-20 glyceryl stearate, PEG-20 glyceryl oleate, PEG-30 glyceryl oleate, PEG-3 castor oil, PEG-5, 9, and 16 castor oil, PEG-20 castor oil , PEG-23 castor oil, PEG-30 castor oil, PEG-35 castor oil, PEG-38 castor oil, PEG-40 castor oil, PEG-50 castor oil, PEG-56 castor oil, PEG-60 castor oil, PEG-100 castor oil, PEG-200 castor oil, PEG-5 hydrogenated castor oil, PEG-7 hydrogenated castor oil, PEG-10 hydrogenated castor oil, PEG-20 hydrogenated castor oil, PEG-25 hydrogenated castor oil, PEG-30 hydrogenated castor oil, PEG-40 hydrogenated castor oil, PEG-45 hydrogenated castor oil, PEG-50 hydrogenated castor oil, PEG-60 hydrogenated castor oil, PEG-80 hydrogenated castor oil, PEG-100 hydrogenated castor oil, PEG-6 corn oil, PEG-6 almond oil, PEG-6 apricot kernel oil, PEG-6 olive oil, PEG-6 peanut oil, PEG-6 hydrogenated palm kernel oil, PEG-6 palm kernel oil, PEG-6 triolein, PEG-8 corn oil, PEG-20 corn glycerides, PEG-20 almond glycerides, PEG-25 trioleate, PEG-40 palm kernel oil, PEG-60 corn glycerides, PEG-60 almond glycerides, PEG-4 caprylic/capric triglyceride, PEG-8 caprylic/capric glycerides, PEG-6 caprylic/capric glycerides, lauroyl macrogol-32 glyceride, stearoyl macrogol glyceride, pentaerythrityl tetraisostearate, pentaerythrityl distearate, pentaerythrityl tetraoleate, pentaerythrityl tetrastearate, pentaerythrityl tetracaprylate/tetracaprate, pentaerythrityl tetraoctanoate, polyglyceryl-2 stearate, polyglyceryl-2 oleate, polyglyceryl-2 isostearate, polyglyceryl-3 oleate, polyglyceryl-4 oleate, polyglyceryl-4 stearate, polyglyceryl-6 oleate, polyglyceryl-10 laurate, polyglyceryl-10 oleate, polyglyceryl-10 stearate, polyglyceryl-6 ricinoleate, polyglyceryl-10 linoleate, polyglyceryl-6 pentaoleate, polyglyceryl-3 dioleate, polyglyceryl-3 distearate, polyglyceryl-4 pentaoleate, polyglyceryl-6 dioleate, polyglyceryl-2 dioleate, polyglyceryl-10 trioleate, polyglyceryl-10 pentaoleate, polyglyceryl-10 septaoleate, polyglyceryl-10 tetraoleate, polyglyceryl-10 decaisostearate, polyglyceryl-101 decaoleate, polyglyceryl-10 mono or dioleate, polyglyceryl polyricinoleate, propylene glycol monocaprylate, propylene glycol monolaurate, propylene glycol oleate, propylene glycol myristate, propylene glycol monostearate, propylene glycol hydroxy stearate, propylene glycol ricinoleate, propylene glycol isostearate, propylene glycol monooleate, propylene glycol dicaprylate/dicaprate, propylene glycol dioctanoate, propylene glycol caprylate/caprate, propylene glycol dilaurate, propylene glycol distearate, propylene glycol dicaprylate, propylene glycol dicaprate, oleic acid, stearic acid, monopalmitolein, monoelaidin, monocaproin, monocaprylin, monocaprin, monolaurin, glyceryl monomyristate, glyceryl monooleateglyceryl monooleate, glycerol monooleate/linoleate, glycerol monolinoleate, glyceryl ricinoleate, glyceryl monolaurate, glycerol monopalmitate, glycerol monostearate, glyceryl mono- or dioleate, glyceryl palmitic/stearic, glyceryl acetate, glyceryl laurate, glyceryl citrate/lactate/oleate/ linoleate, glyceryl caprylate, glyceryl caprylate/caprate, caprylic acid mono- or diglycerides, caprylic/capric glycerides, mono- or diacetylated monoglycerides, glyceryl monostearate, lactic acid esters of mono- or diglycerides, dicaproin, dicaprin, dioctanoin, dimyristin, dipalmitin, distearin, glyceryl dilaurate, glyceryl dioleate, glycerol esters of fatty acids , dipalmitolein, 1,2 and 1,3-diolein, dielaidin, dilinoleincholesterol, sitosterol, lanosterol, PEG-24 cholesterol ether, PEG-30 cholestanol, phytosterol, PEG-25 phyto sterol, PEG-5 soya sterol, PEG-10 soya sterol, PEG-20 soya sterol, PEG-30 soya sterol, PEG-10 sorbitan laurate, PEG-20 sorbitan monolaurate, PEG-4 sorbitan monolaurate, PEG-80 sorbitan monolaurate, PEG-6 sorbitan monolaurate, PEG-20 sorbitan monopalmitate, PEG-20 sorbitan monostearate, PEG-4 sorbitan monostearate, PEG-8 sorbitan monostearate, PEG-6 sorbitan monostearate, PEG-20 sorbitan tristearate, PEG-6 sorbitan tetrastearate, PEG-60 sorbitan tetrastearate, PEG-5 sorbitan monooleate, PEG-6 sorbitan monooleate, PEG-20 sorbitan monooleate, PEG-40 sorbitan oleate, PEG-20 sorbitan trioleate , PEG-6 sorbitan tetraoleate, PEG-30 sorbitan tetraoleate, PEG-40 sorbitan tetraoleate, PEG-20 sorbitan monoisostearate, PEG sorbitol hexaoleate, PEG-6 sorbitol hexastearate, PEG-2 oleyl ether, PEG-3 oleyl ether, PEG-5 oleyl ether, PEG-10 oleyl ether, PEG-20 oleyl ether, PEG-4 lauryl ether, PEG-9 lauryl ether, PEG-23 lauryl ether, PEG-2 cetyl ether, PEG-10 cetyl ether, PEG-20 cetyl ether, PEG-2 stearyl ether, PEG-10 stearyl ether, PEG-20 stearyl ether, PEG-100 stearyl ether, sucrose distearate, sucrose distearate/monostearate, sucrose dipalmitate, sucrose monostearate , sucrose monopalmitate , sucrose monolaurate, poloxamer 105, poloxamer 108, poloxamer 122, poloxamer 123, poloxamer 124, poloxamer 181, poloxamer 182, poloxamer 183, poloxamer 184, poloxamer 185, poloxamer 188, poloxamer 212, poloxamer 215, poloxamer 217, poloxamer 231, poloxamer 234, poloxamer 235, poloxamer 237, poloxamer 238, poloxamer 282, poloxamer 284, poloxamer 288, poloxamer 331, poloxamer 333, poloxamer 334, poloxamer 335, poloxamer 338, poloxamer 401, poloxamer 402, poloxamer 403, poloxamer 407, sorbitan monolaurate, sorbitan monopalmitate, sorbitan monooleate, sorbitan monostearate, sorbitan trioleate, sorbitan sesquioleate, sorbitan tristearate, sorbitan monoisostearate, sorbitan sesquistearate, ethyl oleate, isopropyl myristate, isopropyl palmitate, ethyl linoleate, isopropyl linoleate, sodium caproate, sodium caprylate, sodium caprate, sodium laurate, sodium myristate, sodium myristolate, sodium palmitate, sodium palmitoleate, sodium oleate, sodium ricinoleate, sodium linoleate, sodium linolenate, sodium stearate, sodium lauryl sulfate , sodium tetradecyl sulfate, sodium lauryl sarcosinate, sodium dioctyl sulfosuccinate, sodium cholate, sodium taurocholate, sodium glycocholate, sodium deoxycholate, sodium taurodeoxycholate, sodium glycodeoxycholate, sodium ursodeoxycholate, sodium chenodeoxycholate, sodium taurochenodeoxycholate, sodium glyco cheno deoxycholate, sodium cholylsarcosinate, sodium n-methyl taurocholate, sodium lithocholate, egg/soy lecithin lyso egg/soy lecithin, hydroxylated lecithin, lysophosphatidylcholine, cardiolipin, sphingomyelin, phosphatidylcholine, phosphatidyl ethanolamine, phosphatidic acid, phosphatidyl glycerol, phosphatidyl serine, diethanolammonium polyoxyethylene-10 oleyl ether phosphate, esterification products of fatty alcohols or fatty alcohol ethoxylates with phosphoric acid or anhydride, ether carboxylates (by oxidation of terminal OH group of fatty alcohol ethoxylates), succinylated monoglycerides, sodium stearyl fumarate, stearoyl propylene glycol hydrogen succinate, mono/diacetylated tartaric acid esters of mono- or diglycerides, citric acid esters of mono- or diglycerides, glyceryl-lacto esters of fatty lactylic esters of fatty acids, calcium/sodium stearoyl-2-lactylate, calcium/sodium stearoyl lactylate, alginate salts, propylene glycol alginate, ethoxylated alkyl sulfates, alkyl benzene sulfones, α-olefin sulfonates, acyl isethionates, acyl taurates, alkyl glyceryl ether sulfonates, octyl sulfosuccinate disodium, disodium undecylenamideo-MEA-sulfosuccinate, lauroyl carnitine, palmitoyl carnitine, myristoyl carnitine, hexadecyl triammonium bromide, decyl trimethyl ammonium bromide, cetyl trimethyl ammonium bromide, dodecyl ammonium chloride, alkyl benzyldimethylammonium salts, diisobutyl phenoxyethoxydimethyl benzylammonium salts, alkylpyridinium salts, lauryl betaine, polyoxyethylene-15 coconut amine, or any mixture or combination thereof. Particularly good results were obtained with hexadecyl trimethyl ammonium chloride, polyvinyl alcohol, poly(ethylene oxide) of different molecular weights and poly(p-phenylene oxide) of different molecular weight. Advantageously may hexadecyl trimethyl ammonium chloride, polyvinyl alcohol , poly(ethylene oxide) of different molecular weights and poly(p-phenylene oxide) of different molecular weight be used in a process of coating clarithromycin, ketoprofen, dipyridamole or pimobendan. The choice of optimal anti-aggregating agent depends on the strength of its interaction with the surface of the API. When the physical binding to the surface of the API is strong, anti-aggregating agent can provide the nucleation sites for the coating.

During the process, the suspension comprising the pharmaceutically active ingredient can preferrably be stirred to further prevent that the suspended particles sediment. In addition, constant stirring of the solvent helps to provide homogenous ion concentrations throughout the suspension, thus preventing the ion concentrations to locally exceed the solubility limit, particularly at the place, where ions or counter ions are added to the suspension with the particles. Controlling the stirring of the solvent allows the process to be even more beneficial. However, the stirring should not provide too much shear stress and turbulent flow of the solvent, as this builds new nucleation cites and reduces yield. The best result is obtained when the solvent is stirred at the preferred rate of 100 to 1000 rpm. For example, the best way of stirring the solvent is by rotating the vessel, where reaction takes place, without any paddle. Second best is mixing the solvent with a paddle mixer. It is understood that the shape of the paddle also influences how much stress it induces in the solvent.

According to the process of the present invention, the resulting coated particle can be isolated by centrifuging, decanting or filtrating the suspension. The obtained particles have preferably a particle size of more than 0.5 µm, preferably more than 1 µm, even more preferably of more than 10 µm, further preferred of more than 100 µm. Said particles have a coating of carbonate salt(s), phosphate salt(s) or mixtures thereof that extends over the complete surface of the particle. The size of the coated particles can be controlled by adjusting the pH of the solvent, the time taken for the precipitation process and the stirring rate.

It is additionally preferred that the particle(s) coated with (a) carbonate salt(s), phosphate salt(s), or mixtures thereof comprising an pharmaceutically active ingredient(s), comprise(s) an additional coating layer directly under the coating comprising the carbonate salt(s), phosphate salt(s), or mixtures thereof. The additional coating layer preferably comprises more than 50 % w/w of surfactants, organic salts, saccharides, synthetic polymers, silica derivatives, wax or salts or mixtures thereof, preferably more than 70 % w/w, yet more preferably more than 90 % w/w, based on the weight of the layer. Particularly, the additional layer comprises more than 95% w/w of surfactants, saccharides, synthetic polymers, organic salts, silica derivatives, waxes, or salts or mixtures thereof. The silica derivatives, silicon dioxides and surfactants derivatized with organic compounds are defined as above. Using the aforementioned preferred amounts of coating ingredients facilitates deposition of precipitated carbonate or phosphate salts during the subsequent precipitation.

The amounts of other compounds present in the layer depend on the method of obtaining the layer. If the layer is prepared by the precipitation technique according to the invention, involving suspending the particles in a solvent comprising the compound and removing the solvent, it can consist essentially of only the compound, however, it can also include other compounds if said other compounds are dissolved or suspended in the solvent and coprecipitated or trapped when making the layer. If the layer is prepared by dip coating or spray drying technique it can comprise other compounds like plasticizers or antioxidants that are customary employed in said techniques. The intermediate layer also gives the particles extra stiffness and prevents the coating from falling off.

The present invention also relates to coated particles prepared by using the process for coating particles as described herein.

The present invention relates to particle(s) comprising one or more pharmaceutically active ingredients (APIs), wherein the particle(s) is/are coated with a coating comprising a carbonate salt, phosphate salt, or mixture thereof. Preferably, the particle size of the particle with the coating comprising a carbonate salt, phosphate salt, or mixture thereof (particle coated according to the invention without further optional coatings) is preferably equal to or more than 0.5 µm. Suitable API(s) and materials which can be used for the coating are described above. In pharmaceutical compositions comprising a plurality of particles, the mean particle size d(90) of the particles with the coating comprising a carbonate salt, phosphate salt, or mixture thereof can preferably be equal to or more than 0.5 µm, further preferred, the mean particle size d(90) can be in a range from 0.1 µm to 10 mm, further preferred in a range from 0.2 µm to 4 mm, more preferably in a range from 0.2 µm to 1 mm and most preferably in a range from 0.5 µm to 1 mm.

The present invention also relates to particle(s) comprising a pharmaceutically active ingredient (API), wherein the particle(s) is/are coated with a coating comprising a carbonate salt, phosphate salt, or mixture thereof, and wherein the coated particle additionally comprising one or more additional layers between the particle and the coating comprising a carbonate salt, phosphate salt, or mixture thereof (preferably, the layer is underneath said coating), wherein at least one of the additional layers comprises, preferably in an amount of equal to or more than 50 % w/w, at least one compound selected from the group consisting of surfactants, organic salts, saccharides, synthetic polymers, silica derivatives, wax or salts or mixtures thereof. Preferred materials for the additional layers and suitable amounts are described above.

The present invention also refers to pharmaceutical compositions comprising one or more of the coated particles. Pharmaceutical compositions according to the present invention can be prepared by any known method which is suitable for incorporating particles containing the API(s) in a pharmaceutical composition, e.g. filling into capsules or compressing into tablets together with suitable pharmaceutical excipients.

A pharmaceutical composition can preferably be in the form of a dosage form. The skilled person will know which further excipients are suitable for preparing specific dosage form such as granulates, pellets, microcapsules, tablets, capsules, suppositories, or dosage forms.Dosage forms according to the invention can be further modified or combined with another dosage form. The resulting dosage form can for example be a tablet, which can be a film coated tablet, bilayer or multilayer tablet, mantle coated tablet; uncoated or coated pellet, capsule comprising tablets, powder, granulate, or tablet or mixture thereof.

The pharmaceutical composition may be directly compressed or firstly granulated and then compressedin a tablet press to obtain tablets. Double- or multilayer-mode can be used for the tablet press to prepare double- or multi-layered tablets. Also, a tablet press can be used to obtain mantle coating. Film coating, on the other hand, can be prepared by dissolving or dispersing the pharmaceutical composition in a suitable vehicle for film coating and depositing the vehicle on the cores. Various spheronization or extrusion techniques can be employed to prepare pellets. Optionally, each dosage form is further mantle coated or film coated. Film coating can be prepared by dissolving or dispersing suitable polymer for coating and if necessary plasticizers, stabilizers (antioxidants, acidic or basic agents, etc.) or the like in a vehicle and depositing the vehicle on the dosage form. The process can be done for example by spraying technique or dip coating. Example of the apparatus used is a coating pan. Mantle coating can be achieved by compacting the pharmaceutical formulation around the dosage form. Furthermore, the obtained coated particles can be directly used as a pharmaceutical composition in the form of a suspension, however, in this case the bad taste of the coated particles should be first masked. The process according to the invention can also be easily applied for taste masking in a simple and well controlled manner.

Furthermore, the suspension of particles comprising the pharmaceutically active ingredient(s) can be directly used as a pharmaceutical composition. If desired, the suspension can be supplemented with pharmaceutically acceptable excipients like flavouring agent, conservant, coloring agent, viscosity modifier or other additives for achieving specific properties of the suspension, or another pharmaceutically active ingredient. Further variations of preparing liquid pharmaceutical compositions will be apparent to the skilled person.

A suspension which has been prepared according to the process of the present invention can also be transformed into a solid or semi solid pharmaceutical composition by removing the solvent. This can be simply done by partly or completely drying the suspension. One practical variation of removing the solvent is to spray-dry the prepared suspension while depositing the particles on the inert cores of pharmaceutically acceptable excipient like lactose, or cores like granulate, pellets, tablets, comprising (the) pharmaceutically active ingredient(s), which can be the same or different to the one in the particles. Dried suspension can be used alone as a powder, granulate or as a core in further coating techniques. Dried suspension can be optionally mixed with another pharmaceutically active ingredient or excipient to achieve desired characteristics for transforming or molding the prepared suspension to solid pharmaceutical composition or dosage form. Suitable excipients are any conventionally used pharmaceutically acceptable excipients. For example, the excipients are fillers (lactose, mannitol, cellulose derivatives, sucrose, etc.), disintegrators (crosscarmellose sodium, calcium carbonate, carboxymethylcellulose calcium etc.), binders (polyvinylpyrrolidone, etc.), lubricants (magnesium stearate, stearic acid, silicium dioxide, etc.), fragrants, colorants (titanium dioxide, etc.), sweeteners (saccharine, etc.) or coating polymers (hydroxypropylmethylcellulose, etc.), vehicles (water, organic solvents, etc.).

The coated particles according to the present invention can be optionally mixed with suitable excipients and tabletted or filled into capsules. One specific use of the present invention is to use the process for taste masking of the pharmaceutically active ingredient and then preparing a fast disintegrating or oral dispersible tablets from it. Normally, fast disintegrating or oral dispersible tablets can be prepared by freeze drying of a suspension or mixing it with a superdisintegrant and tabletting it in the tabletting machine. The coating consisting mainly of the carbonate salt(s), phosphate salt(s) or mixtures thereof which do not dissolve in the mouth cavity and thus have the ability to mask the taste of the pharmaceutically active ingredient, but dissolve immediately as the particle proceeds to the stomach.

Specifically preferred are pharmaceutical compositions that comprise particles obtained by the present process of the particle size of more than 0.5 µm, preferably more than 1 µm. In yet another preferred embodiment, the pharmaceutical composition contains particles with an intermediate coating laying just under the carbonate salt or phosphate salt coating comprising the abovementioned excipients that promote deposition of the carbonate salt(s) or phosphate salt(s) on the surface of the particles. The particles according to the present invention having additional coatings beneath the coating comprising carbonate and phosphate salts are advantageous because their properties can easily be manipulated and their mechanical properties can be advantageously applied in the process of preparing pharmaceutical compositions. In addition, said advantages are well reflected in the prepared pharmaceutical composition in terms of better stability or handling features.

The process according to any embodiment of the present invention, the coated particles and pharmaceutical compositions are suitable for preparing a medicament.

The present invention also refers to the use of the process according to the present invention, or of the coated particle according to the present invention, or of the pharmaceutical composition according to the present invention for preparing a medicament.

The present invention also refers to the use of the process according to the present invention for taste masking of a pharmaceutically active ingredient.

### Brief description of the figures

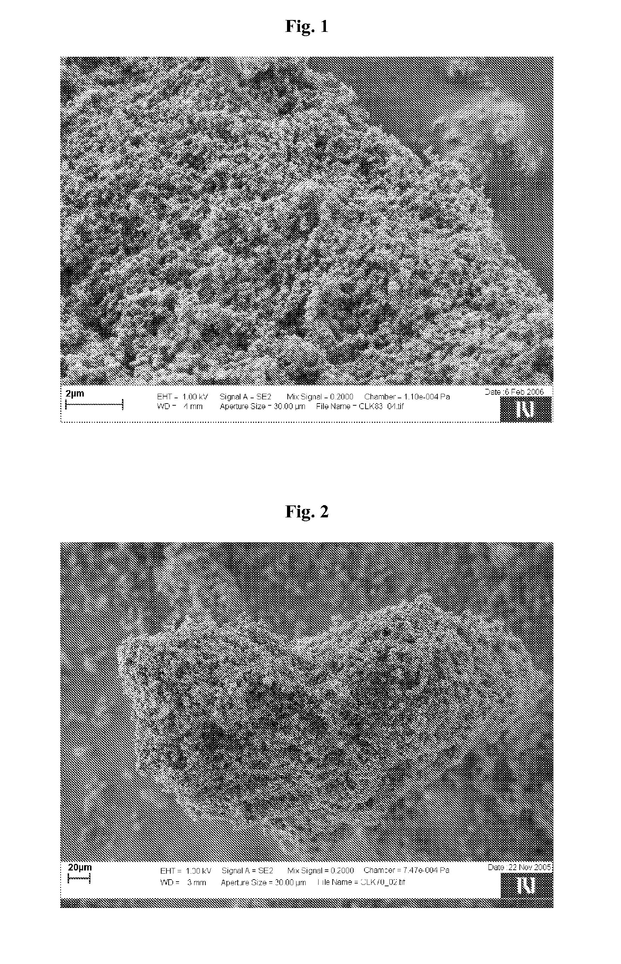

Fig. 1: Image of the coated particle surface obtained by the process as described in example 1 taken by scanning electron microscope.
Fig. 2: Image of the particle obtained by the process as described in example 2 taken by scanning electron microscope.
Fig. 3: Image of the particles obtained by the process as described in example 4 taken by scanning electron microscope, displaying a particle having the size of 0.5 µm.
Fig. 4: Image taken by scanning electron microscope showing unsatisfactory coating on the particle obtained by the process, wherein the ion and counter ion were combined over less than 1 minute.
Fig. 5: Study as described in example 7 of the ketoprofen release from the coated particles of example 6 (TLK148) in comparison with ketoprofen release from uncoated ketoprofen particles.

The following examples merely illustrate the present invention and they should not be considered as limiting the scope of the invention in any way, as these examples, modifications and other equivalents thereof will become apparent to those versed in the art in the light of the present disclosure, examples and the accompanying claims.

### Example 1

A gelatin was dissolved in water in a concentration of 2 g per 1 liter by heating the mixture at 60 °C. 2 g of cooled gelatin solution were supplemented with 0.5 g of clarithromycin. The prepared suspension was homogenized using ultrasound bath for 10 minutes and then centrifuged for 3 minutes at 3000 rpm. The supernatant was discarded. The sediment was resuspended in 100 ml of water and homogenized. While gently mixing the suspension, 20 ml of a solution containing phosphate ions in a concentration of 0.2 mol/l, which was prepared by diluting phosphorous acid, and 20 ml of a solution containing 0.32 mol/I of calcium ions and 0.025 mol/I of magnesium ions, which was prepared by dissolving CaCl₂*6H₂O and MgCl₂ in water, were simultaneously added to the clarithromycin suspension at the rate of 0.3 ml/min for each solution. During admixing solutions to the suspension, the pH was monitored and adjusted to 9 by sodium carbonate solution (1 M). After 70 minutes the suspension was filtered and filtrate dried overnight at 40 °C.

### Example 2

0.2 ml of tetraethyl orthosilicate and 10 ml of isopropanol were added to 100 ml of water. The mixture was homogenized by mixing. Afterwards, the mixture was supplemented with 0.5 g of clarithromycin and further homogenized for 10 minutes by using an ultrasound bath. To the obtained suspension further solutions as described in example 1 were added. In this case, the pH was adjusted to 8.5. After the completion of the precipitation, the prepared particles were separated from the mixture and dried overnight at 40 °C.

### Example 3

12 g of a zinc-calcium solution comprising 0.67 mol/I calcium ions and 0.15 mol/I zinc ions, which was prepared by dissolving CaCl₂*6H₂O and Zn(CH₃COO)₂*2H₂O in water, was supplemented with 1 g of ketoprofen and homogenized for 1 minute by using ultrasound bath in order to prepare a suspension. Then, 1.2 ml of a solution containing phosphate ions in a concentration of 5 mol/I were added to the suspension at a rate of 0.02 ml/min. Constant pH of 4 was achieved by sodium carbonate solution (1 M). After 10 minutes, the mixing was completed and the prepared particles were filtered and dried overnight at 40 °C.

### Example 4

1 g of API was added to 10 g of 0.002 M aqueous HCl. Subsequently, 5 g of the gelatin solution as prepared in example 1 was added to the suspension containing ketoprofen. The resulting suspension was homogenized using ultrasound bath for 10 minutes. Then, the particles were separated after centrifuging the suspension for 3 minutes at 3000 rpm. Particles were added to 12 g of a zinc-calcium solution, which was prepared from CaCl₂*6H₂O and Zn(CH₃COO)₂*2H₂O in water to obtain 0.67 mol/I of calcium ions and 0.15 mol/I of zinc ions, and homogenized for 1 minute. Then, 1.2 ml of a solution containing phosphate ions in a concentration of 5 mol/I were added to the suspension at a rate of 0.02 ml/min. The pH value of the suspension was kept at 4 by adding sodium carbonate solution (1 M). 10 minutes after adding the phosphate solution was completed, and the prepared particles were filtered and dried overnight at 40 °C.

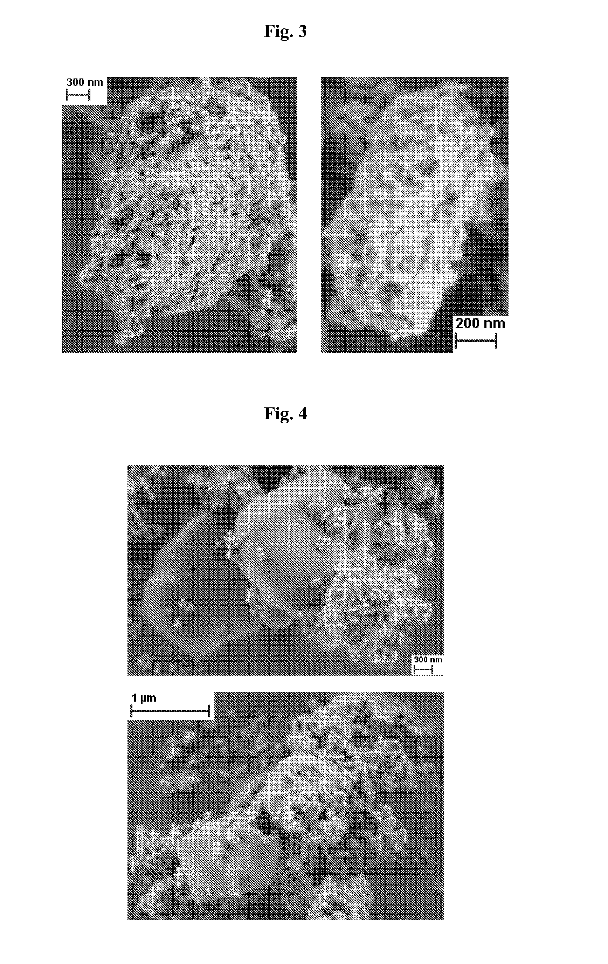

Obtained particles are depicted on Fig 3 and clearly show that with the process of the present invention even coated particles of particle size of around 0.5 µm can be prepared.

### Example 5

12 g of a zinc-calcium solution comprising 0.67 mol/I calcium ions and 0.3 mol/I zinc ions, which was prepared by dissolving Ca(CH3COO)2*H2O and Zn(CH3COO)2*2H2O in water, was supplemented with 1 g of ketoprofen and homogenized for 1 minute by using ultrasound bath in order to prepare a suspension. Then, 6 ml of a solution containing phosphate ions in a concentration of 5 mol/I were added fast (in few seconds) to the suspension. pH value of 4 was achieved by adding a proper amount of sodium carbonate solution (1 M). After 10 minutes the prepared particles were filtered and dried overnight at 40 °C. The prepared particles are shown on Fig 4. The precipitation of salts was lead over less than 1 minute, which resulted in deposition of salts only to parts of particle surface.

### Example 6

In the first step of preparation, 1 g of ketoprofen was dispersed in 10 g of water solution of HCl (0.06 g) with the pH of 4. After 5 minutes of mixing with the magnetic stirrer at 100 rpm, 0.01 g of glutamic acid was added to the solution, which served as an anti-aggregating agent. The as prepared solution was stirred for another 10 minutes. In the second step mixing of 0.5 g bis-1,2-triethoxysylilethane (BTSE) with 0.5 g of ethanol and 10 g of water was performed. The mixture was homogenized by using either ultrasound for 10 minutes only or ultrasound with an additional heating step in the oven at 50°C for 5 minutes. This step was considered as finished when a milky suspension of primary silicate particles was gathered. In the next the prepared solution in the first step and the suspension in the second were mixed together. The as such prepared suspension was homogenized using ultrasound bath for 10 minutes and then centrifuged for 3 minutes at 3000 rpm. The supernatant was discarded. The sediment was resuspended in 100 ml of water and homogenized. While gently mixing the suspension, 20 ml of a solution containing phosphate ions in a concentration of 0.2 mol/l, which was prepared by diluting phosphorous acid, and 20 ml of a solution containing 0.32 mol/I of calcium ions and 0.025 mol/I of magnesium ions, which was prepared by dissolving CaCl₂*6H₂O and MgCl₂ in water, were simultaneously added to the ketoprofen suspension at the rate of 0.3 ml/min for each solution. The pH value of the suspension was kept at 4 by adding sodium carbonate solution (1 M). 10 minutes after adding the phosphate solution was completed, and the prepared particles were filtered and dried overnight at 40 °C. The coated particles were subjected to thermal gravimetric analysis (TGA), which disclosed that ketoprofen constituted 65.7 % by weight of the coated particle total amount.

### Example 7

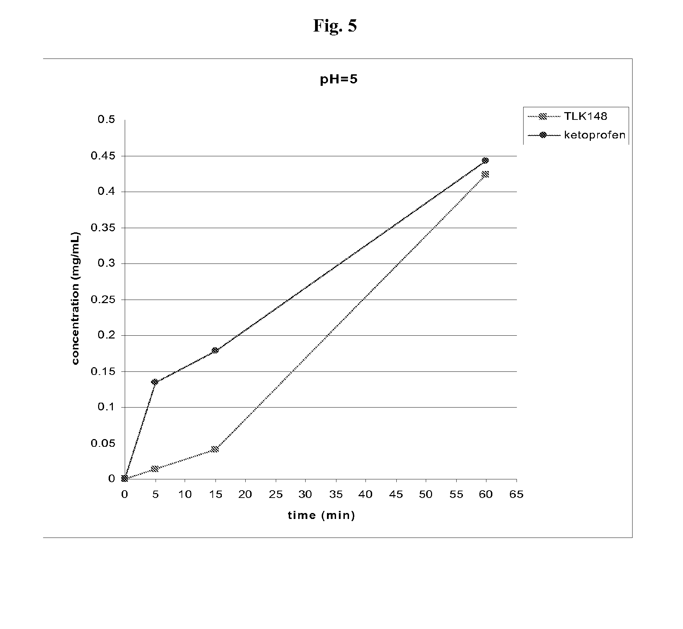

The effect of coating on the release of ketoprofen from the coated particles obtained in example 6 (TLK148) was analyzed in comparison to release from uncoated ketoprofen particles. 325 (±1mg) of particles obtained as described in example 6 were provided in a flask containing 250 ml of phosphate buffer at 25°C and stirred with a magnetic stirrer. Low stirring has been used for first 15 min and increased mixing for the rest of 45 min. pH of the phosphate buffer was set at 5 to simulate oral environment for taste masking application. In parallel, the same steps were done with the corresponding amount of uncoated ketoprofen particles (TGA results were used to arrive at the corresponding amount). Periodically, the samples were taken and analyzed spectroscopically at 259 nm. The results as depicted in Fig. 5 clearly show the delayed release of ketoprofen from the coated particles, which is preferred in taste masking.

### Example 8

A Cetylpiridinum Chloride (CPC) was dissolved in water in a concentration of 0.002 mol per 1 liter..100 g of CPC solution was supplemented with 0.2 g of dipyridamole. The prepared suspension was homogenized using ultrasound bath for 10 minutes. While gently mixing the suspension, 5 ml of a solution containing phosphate ions in a concentration of 0.2 mol/l, which was prepared by diluting phosphorous acid, and 20 ml of a solution containing 0.32 mol/I of calcium ions, which was prepared by dissolving CaCl2*6H20 in water, were simultaneously added to the dipyridamole suspension at the rate of 0.14 ml/min for each solution. During admixing solutions to the suspension, the pH was monitored and adjusted to 9 by sodium carbonate solution (1 M). After 60 minutes the suspension was filtered and filtrate dried overnight at 40 °C.

### Example 9

Pimobendan particles were coated as described in example 8.

### Example 10

Dipyridamole was dispersed in 140 ml water - ammonia solution at pH value 8. Separately 0.6 ml of tetraethyl orthosilicate was diluted in 10 ml of ethanol and then added to dipyridamole dispersion. The mixture was homogenized by using an ultrasound bath for 10 minutes. While gently mixing the suspension, 5 ml of a solution containing phosphate ions in a concentration of 0.2 mol/l, which was prepared by diluting phosphorous acid, and 20 ml of a solution containing 0.32 mol/I of calcium ions, which was prepared by dissolving CaCl2*6H20 in water, were simultaneously added to the dipyridamole suspension at the rate of 0.14 ml/min for each solution. During admixing solutions to the suspension, the pH was monitored and adjusted to 9 by sodium carbonate solution (1 M). After 60 minutes the suspension was filtered and filtrate dried overnight at 40 °C.

### Example 11

Example 10 was repeated with pimobendan.

## Claims

1. A process for coating a particle comprising a pharmaceutically active ingredient (API) comprising the steps of:
a) providing a composition comprising carbonate ions, phosphate ions, or a mixture thereof,
b) providing particle(s) comprising an API, and
c) precipitating a carbonate salt, a phosphate salt or a mixture thereof onto the particle(s) comprising the API, wherein the precipitation is carried out in a suspension having a pH of 4 or higher.

2. A process for coating a particle comprising a pharmaceutically active ingredient (API), comprising the steps of:
a) providing a composition comprising carbonate ions, phosphate ions, or a mixture thereof,
b) providing particle(s) comprising an API, and
c) precipitating a carbonate salt, a phosphate salt or a mixture thereof onto the particle(s) comprising the API, and wherein the precipitation is achieved by gradually combining the carbonate ions, the phosphate ions or the mixture thereof with counter ions in the presence of the particle(s) comprising the API.

3. The process according to claim 2, wherein the gradually combining is achieved over a period of at least 1 minute.

4. The process according to any of claims 1 to 3,
wherein precipitation is achieved by combining a suspension comprising the particle(s) comprising the API and the composition of step a) with a solvent containing counter ions for the carbonate ions and/or phosphate ions, or by combining a suspension comprising the particle comprising the API and counter ions for the carbonate ions and/or phosphate ions with a solvent containing the composition of step a), or by combining a suspension comprising the API with a solvent containing the composition of step a) and another solvent containing the counter ions for the carbonate ions and/or phosphate ions.

5. The process according to any one of the preceding claims, wherein the amount of the particle(s) comprising pharmaceutically active ingredient in the suspension during precipitation is in a range of from 0.1 % to 50 % w/w based on the total amount of solvent(s) used in the process.

6. The process according to any one of the preceding claims, wherein the total amount of the carbonate ions, phosphate ions or mixture thereof is from 0.002 to 5 mol/L based on the total amount of solvent used in the process.

7. The process according to any of claims 2 to 6, wherein the total amount of counter ions for precipitating the carbonate salt(s), phosphate salt(s), or mixture thereof is from 0.002 to 5 mol/L based on the total amount of solvent used in the process.

8. The process according to any one of the preceding claims, wherein the carbonate ions, phosphate ions or mixture thereof are provided by one or more compounds selected from the group consisting of phosphorous acid, phosphorous acid water soluble salts, carbonic acid, carbonic acid water soluble salts, carbon dioxide, and mixtures thereof.

9. The process according to any of claims 4 to 8, wherein the counter ions for precipitating a carbonate salt, phosphate salt, or mixture thereof are provided by one or more compounds selected from the group consisting of calcium salts, magnesium salts, zinc salts, barium salts, strontium salts, copper salts, iron salts and mixtures thereof; preferably from the group consisting of calcium salts, magnesium salts, zinc salts, barium salts and mixtures thereof; more preferably from the group consisting of calcium salts, magnesium salts, zinc salts and mixtures thereof.

10. The process according to any of claims 2 to 9, wherein the pH of the suspension comprising particle(s) comprising API during the precipitation is 4 or higher.

11. The process according to any one of the preceding claims, wherein the suspension comprising the particle(s) comprising API further comprises an anti-aggregating agent.

12. The process according to any one of the previous claims, further comprising a step of precipitating a compound selected from the group consisting of surfactants, organic salts, saccharides, synthetic polymers, silica derivatives, waxes and salts, or a mixture thereof, on the surface of the particle comprising an API prior to precipitating the carbonate salt, the phosphate salt or mixtures thereof.

13. The process according to any of the preceding claims, further comprising a step of preparing a pharmaceutical composition.

14. A particle comprising a pharmaceutically active ingredient (API), the particle being coated with a coating comprising a carbonate salt, phosphate salt, or mixture thereof.

15. The particle according to claim 14, wherein the particle size of the particle with said coating is at least 0.5 µm.

16. The particle according to claim 14, wherein the particle additionally comprises a layer underneath said coating, wherein the layer comprises, preferably in an amount of equal to or more than 50 % w/w, at least one compound selected from the group consisting of surfactants, organic salts, saccharides, synthetic polymers, silica derivatives, waxes or salts or mixtures thereof.

17. A pharmaceutical composition comprising one or mre particle(s) as defined in any of claims 14 to 16.

18. Use of the process according to any one of claims 1 to 13, or of the coated particle according to claims 14 to 16, or of the pharmaceutical composition according to claim 17 for preparing a medicament.

19. Use of the process according to any one of claims 1-13 for taste masking of a pharmaceutically active ingredient.
